(19) **Europäisches Patentamt / European Patent Office / Office européen des brevets**

(11) **EP 3 661 478 B1**

(12) **FASCICULE DE BREVET EUROPEEN**

(45) Date de publication et mention de la délivrance du brevet:
**05.05.2021 Bulletin 2021/18**

(21) Numéro de dépôt: **18752834.4**

(22) Date de dépôt: **31.07.2018**

(51) Int Cl.:
*A61K 8/34* (2006.01)     *A61K 8/60* (2006.01)
*A61K 8/06* (2006.01)     *A61Q 1/14* (2006.01)
*A61Q 17/04* (2006.01)     *A61Q 19/00* (2006.01)

(86) Numéro de dépôt international:
**PCT/FR2018/051961**

(87) Numéro de publication internationale:
**WO 2019/025715 (07.02.2019 Gazette 2019/06)**

(54) **NOUVELLE COMPOSITION TENSIOACTIVE, SON PROCÉDÉ DE PRÉPARATION, ET SON UTILISATION COMME ÉMULSIONNANT POUR PRÉPARER DES ÉMULSIONS TOPIQUES HUILE-DANS-EAU**

NEUE TENSIDZUSAMMENSETZUNG, VERFAHREN ZU IHRER HERSTELLUNG UND IHRE VERWENDUNG ALS EMULGATOR ZUR HERSTELLUNG TOPISCHER ÖL-IN-WASSER-EMULSIONEN

NOVEL SURFACTANT COMPOSITION, PRODUCTION METHOD THEREOF, AND USE OF SAME AS AN EMULSIFIER FOR THE PRODUCTION OF TOPICAL OIL-IN-WATER EMULSIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.08.2017 FR 1757405**

(43) Date de publication de la demande:
**10.06.2020 Bulletin 2020/24**

(73) Titulaire: **Société d'Exploitation de Produits pour les Industries Chimiques SEPPIC**
**75321 Paris Cedex 7 (FR)**

(72) Inventeurs:
• **CLEMENCEAU, Florence**
**81100 Castres (FR)**
• **DACOSTA, Georges**
**81710 Saix (FR)**
• **GUILBOT, Jérôme**
**81100 Castres (FR)**
• **TAILLEBOIS, Cécile**
**FR-81100 CASTRES (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
WO-A1-96/37285     WO-A1-2007/113440
FR-A1- 2 959 140     US-A1- 2001 018 424

**Description**

La présente invention concerne une nouvelle composition d'alkyl polyglycosides se présentant sous forme liquide à température ambiante, son procédé de préparation, son utilisation comme agent émulsionnant, notamment pour préparer des émulsions de type huile-dans-eau à usage topique. L'invention trouve application préférentiellement dans le domaine cosmétique et dermocosmétique, dans le domaine dermopharmaceutique et pharmaceutique, notamment dans l'utilisation pour le soin, la protection et le nettoyage de la peau, des cheveux et du cuir chevelu, mais également dans le domaine de l'industrie textile, par exemple pour le traitement de fibres textiles synthétiques ou naturelles tissées ou tricotées ou encore dans le domaine de l'industrie papetière par exemple pour la fabrication de papier à usage sanitaire ou domestique.

Les émulsions constituent des formes privilégiées de formulations à usage topiques destinées à traiter et/ou à protéger la peau et/ou les muqueuses et/ou le cuir chevelu, et de façon générale des produits de soin corporel, car elles combinent propriétés techniques et sensations positives sur la peau du sujet duquel elles sont appliquées.

Une émulsion est un mélange de deux substances liquides, non miscibles qui en présence d'au moins un agent tensioactif et après agitation conduit à une forme liquide macroscopiquement homogène mais microscopiquement hétérogène et qui est une dispersion d'une des deux phases dans l'autre phase sous forme de fines gouttelettes. Un tel agent de surface permettant de conserver la stabilité de la dispersion obtenue au cours du temps est appelé agent émulsionnant.

Selon la nature de la phase dispersée et de la phase continue, on qualifie ces émulsions d'huile-dans-eau ou d'eau-dans-huile, et pour les émulsions multiples, on qualifie ces émulsions d'huile-dans-eau-dans-huile ou d'eau-dans-huile-dans-eau.

Les agents émulsionnants capables de conférer la stabilité aux émulsions sont généralement des composés amphiphiles, dont la structure moléculaire comprend une partie hydrophile et une partie lipophile, de nature anionique, cationique, amphotère ou non-ionique. Pour une application topique de l'émulsion, on a depuis longtemps préféré les tensioactifs de nature non-ionique ont été préférés car ils sont peu irritants. Il y a par exemple les alcools gras, généralement comportant de 16 à 22 atomes de carbone, polyéthoxylés avec une stœchiométrie précise d'oxyde d'éthylène pour apporter le degré d'hydrophilie souhaité. Un tel exemple est la CIRE de LANOL CTO™, résultant l'éthoxylation de mélanges d'alcools gras comportant 16 et 18 atomes de carbone par 20 à 35 moles d'éthylène.

Le durcissement des contraintes réglementaires relatives à la présence potentielle résiduelle de l'oxyde d'éthylène et de son produit de dégradation, le 1,4-dioxane, a conduit les fabricants de tensioactifs à développer de nouvelles structures émulsionnantes de nature non ioniques, comme les alkyl polyglycosides, obtenus à partir de matières premières d'origines renouvelable, comme les alcools gras provenant de la transformation de l'huile de palme et des monosaccharides ou disaccharides de nature réductrice comme par exemple le galactose, le xylose, le glucose, l'arabinose. Lorsque le sucre choisi est le glucose, on nomme les tensioactifs obtenus les « alkyl polyglucosides ».

La demande de brevet français publiée sous le numéro 2 668 080 A1, décrit notamment des compositions auto-émulsionnables comprenant pour 100% de leur masse, de 60% à 90% massique d'un alcool gras, ayant de 12 à 22 atomes de carbone, et de 10% à 40% massique d'un alkyl polyglycoside dont la partie alkyle est identique à celle de l'alcool gras, ainsi que leur utilisation pour la préparer des émulsions de type huile-dans-eau.

Lorsque de telles compositions d'alcools gras et d'alkyl polyglucosides sont obtenues à partir du glucose et d'alcools gras linéaires, saturés, et comportant de 14 à 22 atomes de carbone, en présence d'un milieu catalytique acide adapté, elles présentent une proportion massique alkyl polyglucosides sur alcools gras qui dépend de la stœchiométrie molaire du sucre et des alcools gras retenue au départ. Toutefois, parmi les produits commercialisés, on observe des ratios massiques alkyl polyglucosides sur alcools gras compris entre 5/95 et 30/70, qui présentent un aspect solide, pouvant être mis sous la forme d'écailles ou de perles. De telle compositions sont commercialisées depuis le début des années 90, notamment par la société SEPPIC sous le nom de marque MONTANOV™ et SIMULGREEN™ ; la présence des alcools gras en mélange avec les alkyl polyglucosides, permet notamment à ces agents émulsionnants de type huile-dans-eau, de constituer des précurseurs de cristaux liquides favorisant une meilleure stabilité des émulsions préparées.

Lorsque les compositions d'alcools gras et d'alkyl polyglucosides sont obtenues à partir d'alcools gras linéaires, saturés, et comportant de 14 à 22 atomes de carbone, il est possible d'éliminer partiellement l'excès d'alcools gras de façon à enrichir la fraction d'alkyl polyglucosides à des concentrations massiques comprises entre 50 et 90%. Compte-tenu des points d'ébullition relativement élevés des alcools gras concernés, les technologies « classiques » d'évaporation ne sont pas adaptées et il est alors nécessaire de privilégier des technologies extrêmement performantes comme la technique d'évaporation par la mise en œuvre d'un évaporateur couche mince à court trajet.

Le brevet Américain publié sous le numéro 5,837,831, décrit également un procédé d'extraction à l'aide de fluides supercritiques pour aboutir à des compositions très concentrées en alkyl polyglucosides, généralement à des teneures supérieures ou égales à 90% massique.

La demande internationale publiée sous le numéro WO 96/37285 divulgue des compositions comprenant pour 100% de leur masse de 60% à 90% d'au moins un alcool gras comportant de 12 à 18 atomes de carbone, et de 10% à 40% massique d'au moins un alkyl polyglucoside comportant de 12 à 18 atomes de carbone, ainsi que leur utilisation de telles compositions comme agent émulsionnant destiné à préparer des émulsions huile-dans-eau.

La demande internationale publiée sous le numéro WO 95/13863 décrit des concentrés comportant pour 100% de leur masse de 60% à 90% d'au moins alkyl polyglucoside comportant de 12 à 18 atomes de carbone, et de 10% à 40% massique d'au moins un alcool gras comportant de 12 à 18 atomes de carbone, et leur utilisation agent nacrant dans des émulsions à usage topique.

Les émulsions huile-dans-eau sont préparées par dispersion de ces agents émulsionnants, soit dans l'eau ou dans une phase polaire, soit dans une phase huileuse. Les agents émulsionnants sont donc préalablement portés à une température supérieure à leur point de fusion, dont la valeur se situe entre 60°C et 80°C pour les compositions d'alcools gras et d'alkyl polyglucosides décrites ci-dessus, avant d'être dispersés dans la phase aqueuse ou huileuse de l'émulsion finale. Or cette montée en température constitue un frein à l'expansion commerciale de ces agents émulsionnants à base d'alcools gras et d'alkyl polyglucosides sur des chaînes alkyles comportant de 12 à 18 atomes de carbone.

On a donc cherché à développer des mélanges d'alkyl polyglycosides et d'alcools gras qui soient liquides à température ambiante, à savoir jusqu'à 30°C, pour éviter cette opération de fusion lors de la préparation de l'émulsion finale.

La demande internationale publiée sous le numéro WO 00/56438 A1 divulgue ainsi une composition émulsionnante comprenant 81,9% d'alcool oléique et 18,1% d'alkyl polyglucosides sur alcool oléique, ainsi qu'une composition émulsionnante comprenant 83,9% d'alcool isostéarylique et 16,1% d'alkyl polyglucosides sur alcool isostéarylique.

Cependant, de telles compositions liquides, à cause de leur caractère très lipophile, ne permettent pas la préparation d'émulsions huile-dans-eau suffisamment stables, mais plutôt la préparation d'émulsions eau-dans-huile. D'autre part, ces compositions se caractérisent par une faible solubilité des alkyl polyglucosides dans leur alcool gras, une telle instabilité entraînant l'apparition de dépôt lors du stockage à température ambiante.

La demande de brevet français publiée sous le numéro FR 2 807 435 A1 décrit des compositions émulsionnantes comprenant des alcools de Guerbet comportant de 12 à 20 atomes de carbone et des alkyl polyglycosides à base desdits alcools de Guerbet comportant de 12 à 20 atomes de carbone et de xylose comme sucre réducteur. Ces compositions se présentent sous une forme liquide mais permettent de préparer des émulsions eau-dans-huile au lieu d'émulsions huile-dans-eau.

La demande internationale publiée sous le numéro WO2005/110588 A1 divulgue des mélanges comprenant des alkyl polyglycosides sur chaînes grasses linéaires comportant de 4 à 14 atomes de carbone et des alcools gras ramifiés de Guerbet comportant de 8 à 36 atomes de carbone. De tels mélanges se présentent sous forme liquide et sont adaptés pour préparer des émulsions vaporisables. Cependant, la limitation des longueurs de chaînes des alkyl polyglycosides ne permettent pas d'atteindre les propriétés émulsionnantes requises pour préparer des émulsions huile-dans-eau stables au stockage.

La demande internationale publiée sous le numéro WO 2007/113440 A1 divulgue des mélanges émulsionnants à base d'alcools gras comportant 20 et 22 atomes de carbone optionnellement combinés avec des d'alkyl polyglucosides. Cependant, ces mélanges montrent comme inconvénient de former des grumeaux lors du stockage à température ambiante.

Il subsiste donc un besoin de disposer d'une composition émulsionnante, se présentant sous une forme liquide à température comprise entre 20°C et 30°C, ne comprenant pas de composés obtenus par la mise en œuvre d'oxyde d'éthylène, restant homogène après stockage, permettant de préparer des émulsions huile-dans-eau stables au stockage.

Dans le cadre de leurs recherches sur l'amélioration constante des agents émulsionnants pour préparer des émulsions "huile-dans-eau" qui soient dépourvus des inconvénients cités plus haut, les inventeurs ont mis au point de nouvelles compositions émulsionnantes à base d'alkyl polyglycosides, ainsi qu'un nouveau procédé de préparation d'émulsions huile-dans-eau, permettant de préparer des émulsions stables en présence d' ingrédients thermosensibles et/ou volatils.

Selon un premier aspect, l'invention a pour objet une composition (EM) comprenant pour 100% de sa masse :

**1) -** Une proportion supérieure ou égale à 20% massique et inférieure ou égale à 80% massique d'un agent diluant (D) choisi parmi le 1,3-propanediol, le 1,3-butanediol, le 1,4-butanediol, le 2-méthyl 2,4-pentanediol, le 1,6-hexanediol, le 1,8-octanediol ou un composé de formule (I) :

$$\text{HO-[CH}_2\text{-CH(OH)-CH}_2\text{-O-]}_n\text{-H} \qquad \text{(I)},$$

dans laquelle n représente un nombre entier supérieur ou égal à 1 et inférieur ou égal à 6, ou d'un mélange desdits agents diluants

**2) -** Une proportion supérieure ou égale à 20% massique et inférieure ou égale à 80% massique d'une composition (C) comprenant pour 100% de sa masse :

    **a) -** Une proportion supérieure à 0% et inférieure ou égale à 4% massique d'au moins un alcool de formule (II) :

$$C_mH_{2m+1}\text{-}OH \qquad (II),$$

dans laquelle m représente un nombre entier pair supérieur ou égal à 8 et inférieur ou égal à 18 ;

    **b) -** Une proportion supérieure ou égale à 96% massique et inférieure à 100% massique d'une composition $(C_1)$ comprenant :

        **$b_1$) -** Une proportion supérieure ou égale à 60% massique et inférieure ou égale 90% massique d'une composition $(C_2)$ comprenant pour 100% de sa masse :

        **$b_{1i}$) -** Une proportion supérieure ou égale à 5% massique et inférieure ou égale à 20% massique d'une composition $(C_{21})$ représentée par la formule (III) :

$$R_{21}\text{-}O\text{-}(G_{21})_r\text{-}H \qquad (III),$$

dans laquelle $R_{21}$ représente le radical n-dodécyle, $G_{21}$ représente le reste d'un sucre réducteur et r représente un nombre décimal supérieur ou égal à 1,05 et inférieur ou égal à 5, ladite composition $(C_{21})$ consistant en un mélange des composés de formules $(III_1)$, $(III_2)$, $(III_3)$, $(III_4)$ et $(III_5)$ :

$$R_{21}\text{-}O\text{-}(G_{21})_1\text{-}H \qquad (III_1),$$

$$R_{21}\text{-}O\text{-}(G_{21})_2\text{-}H \qquad (III_2),$$

$$R_{21}\text{-}O\text{-}(G_{21})_3\text{-}H \qquad (III_3),$$

$$R_{21}\text{-}O\text{-}(G_{21})_4\text{-}H \qquad (III_4),$$

$$R_{21}\text{-}O\text{-}(G_{21})_5\text{-}H \qquad (III_5),$$

en des proportions molaires en dits composés de formules $(III_1)$, $(III_2)$, $(III_3)$, $(III_4)$ et $(III_5)$ respectivement égales à $a_1$, $a_2$, $a_3$, $a_4$ et $a_5$, telles que la somme $a_1 + a_2 + a_3 + a_4 + a_5$ est égale à 1, et que la somme $a_1 + 2a_2 + 3a_3 + 4a_4 + 5a_5$ est égale à r ;

        **$b_{1ii}$) -** Une proportion supérieure ou égale à 10% massique et inférieure ou égale à 20% massique d'une composition $(C_{22})$ représentée par la formule (IV) :

$$R_{22}\text{-}O\text{-}(G_{22})_s\text{-}H \qquad (IV),$$

dans laquelle $R_{22}$ représente le radical n-tétradécyle, $G_{22}$ représente le reste d'un sucre réducteur et s représente un nombre décimal supérieur ou égal à 1,05 et inférieur ou égal à 5, ladite composition $(C_{22})$ consistant en un mélange des composés de formules $(IV_1)$, $(IV_2)$, $(IV_3)$, $(IV_4)$ et $(IV_5)$ :

$$R_{22}\text{-}O\text{-}(G_{22})_1\text{-}H \qquad (IV_1),$$

$$R_{22}\text{-}O\text{-}(G_{22})_2\text{-}H \qquad (IV_2),$$

$$R_{22}\text{-}O\text{-}(G_{22})_3\text{-}H \qquad (IV_3),$$

$$R_{22}\text{-}O\text{-}(G_{22})_4\text{-}H \qquad (IV_4),$$

$$R_{22}\text{-}O\text{-}(G_{22})_5\text{-}H \qquad (IV_5),$$

en des proportions molaires en dits composés de formules $(IV_1)$, $(IV_2)$, $(IV_3)$, $(IV_4)$ et $(IV_5)$ respectivement égales à $b_1$, $b_2$, $b_3$, $b_4$ et $b_5$, telles que la somme: $b_1 + b_2 + b_3 + b_4 + b_5$ est égale à 1, et que la somme $b_1 + 2b_2 + 3b_3 + 4b_4 + 5b_5$ est égale à s ;

**b$_{1iii}$)** - Une proportion supérieure ou égale à 25% massique et inférieure ou égale à 40% massique d'une composition (C$_{23}$) représentée par la formule (V) :

$$R_{23}-O-(G_{23})_t-H \qquad (V),$$

dans laquelle R$_{23}$ représente le radical n-octyle, G$_{23}$ représente le reste d'un sucre réducteur et t représente un nombre décimal supérieur ou égal à 1,05 et inférieur ou égal à 5, ladite composition (C$_{23}$) consistant en un mélange des composés de formules (V$_1$), (V$_2$), (V$_3$), (V$_4$) et (V$_5$) :

$$R_{23}-O-(G_{23})_1-H \qquad (V_1),$$

$$R_{23}-O-(G_{23})_2-H \qquad (V_2),$$

$$R_{23}-O-(G_{23})_3-H \qquad (V_3),$$

$$R_{23}-O-(G_{23})_4-H \qquad (V_4),$$

$$R_{23}-O-(G_{23})_5-H \qquad (V_5),$$

en des proportions molaires en dits composés de formules (V$_1$), (V$_2$), (V$_3$), (V$_4$) et (V$_5$) respectivement égales à c$_1$, c$_2$, c$_3$, c$_4$ et c$_5$, telles que la somme: c$_1$+ c$_2$ + c$_3$ + c$_4$ + c$_5$ est égale à 1, et que la somme c$_1$ + 2c$_2$ + 3c$_3$ + 4c$_4$ + 5c$_5$ est égale à t ;

**b$_{1iv}$)** - Une proportion massique supérieure ou égale à 30% massique et inférieure ou égale à 55% massique d'une composition (C24) représentée par la formule (VI) :

$$R_{24}-O-(G_{24})_u-H \qquad (VI),$$

dans laquelle R$_{24}$ représente le radical n-décyle, G$_{24}$ représente le reste d'un sucre réducteur et u représente un nombre décimal supérieur ou égal à 1,05 et inférieur ou égal à 5, ladite composition (C$_{24}$) consistant en un mélange des composés de formules (VI$_1$), (VI$_2$), (VI$_3$), (VI$_4$) et (VI$_5$) :

$$R_{24}-O-(G_{24})_1-H \qquad (VI_1),$$

$$R_{24}-O-(G_{24})_2-H \qquad (VI_2),$$

$$R_{24}-O-(G_{24})_3-H \qquad (VI_3),$$

$$R_{24}-O-(G_{24})_4-H \qquad (VI_4),$$

$$R_{24}-O-(G_{24})_5-H \qquad (VI_5),$$

en dans les proportions molaires en dits composés de formules (VI$_1$), (VI$_2$), (VI$_3$), (VI$_4$) et (VI$_5$) respectivement égales à d$_1$, d$_2$, d$_3$, d$_4$ et d$_5$, telles que la somme: d$_1$ + d$_2$ + d$_3$ + d$_4$ + d$_5$ est égale à 1, et que la somme d$_1$ + 2d$_2$ + 3d$_3$ + 4d$_4$ + 5d$_5$ est égale à u ; étant entendu que la somme des proportions massiques des compositions (C$_{21}$), (C$_{22}$), (C$_{23}$), et (C$_{24}$), est égale à 100% ;

**b$_2$)** - D'une proportion supérieure ou égale à 10% massique et inférieure ou égale à 40% massique d'une composition (C$_3$) comprenant pour 100% de sa masse :

**b$_{2i}$)** - Une proportion supérieure ou égale à 30% massique et inférieure ou égale à 50% massique d'une composition (C$_{31}$) représentée par la formule (VII) :

$$R_{31}-O-(G_{31})_x-H \qquad (VII)$$

dans laquelle R$_{31}$ représente le radical n-hexadécyle, G$_{31}$ représente le reste d'un sucre réducteur et x représente un nombre décimal supérieur ou égal à 1,05 et inférieur ou égal à 5, ladite composition (C$_{31}$) consistant en un mélange des composés de formules (VII$_1$), (VII$_2$), (VII$_3$), (VII$_4$) et (VII$_5$):

$$R_{31}-O-(G_{31})_1-H \qquad (VII_1),$$

$$R_{31}\text{-O-}(G_{31})_2\text{-H} \qquad (VII_2),$$

$$R_{31}\text{-O-}(G_{31})_3\text{-H} \qquad (VII_3),$$

$$R_{31}\text{-O-}(G_{31})_4\text{-H} \qquad (VII_4),$$

$$R_{31}\text{-O-}(G_{31})_5\text{-H} \qquad (VII_5),$$

en des proportions molaires en dits composés de formules $(VII_1)$, $(VII_2)$, $(VII_3)$, $(VII_4)$ et $(VII_5)$ respectivement égales $a'_1$, $a'_2$, $a'_3$, $a'_4$ et $a'5$, telles que la somme $a'_1 + a'_2 + a'_3 + a'_4 + a'_5$ est égale à 1, et que la somme $a'_1 + 2a'_2 + 3a'_3 + 4a'_4 + 5a'_5$ est égale à x ;

$b_{2ii}$) - Une proportion supérieure ou égale à 50% massique et inférieure ou égale à 70% massique d'une composition $(C_{32})$ représentée par la formule (VIII) :

$$R_{32}\text{-O-}(G_{32})_y\text{-H} \qquad (VIII),$$

dans laquelle $R_{32}$ représente le radical n-octadécyle, $G_{32}$ représente le reste d'un sucre réducteur et y représente un nombre décimal supérieur ou égal à 1,05 et inférieur ou égal à 5, ladite composition $(C_{32})$ consistant en un mélange des composés de formules $(VIII_1)$, $(VIII_2)$, $(VIII_3)$, $(VIII_4)$ et $(VIII_5)$ :

$$R_{32}\text{-O-}(G_{32})_1\text{-H} \qquad (VIII_1),$$

$$R_{32}\text{-O-}(G_{32})_2\text{-H} \qquad (VIII_2),$$

$$R_{32}\text{-O-}(G_{32})_3\text{-H} \qquad (VIII_3),$$

$$R_{32}\text{-O-}(G_{32})_4\text{-H} \qquad (VIII_4),$$

$$R_{32}\text{-O-}(G_{32})_5\text{-H} \qquad (VIII_5),$$

En des proportions molaires en dits composés de formules $(VIII_1)$, $(VIII_2)$, $(VIII_3)$, $(VIII_4)$ et $(VIII_5)$ respectivement égales à $b'_1$, $b'_2$, $b'_3$, $b'_4$ et $b'5$, telles que la somme $b'_1 + b'_2 + b'_3 + b'_4 + b'_5$ est égale à 1, et que la somme $b'_1 + 2b'_2 + 3b'_3 + 4b'_4 + 5b'_5$ est égale à y,

étant entendu que la somme des proportions massiques des compositions $(C_{31})$ et $(C_{32})$ est égale à 100%.

Par reste d'un sucre réducteur, on désigne dans la définition des restes $(G_{21})$, $(G_{22})$, $(G_{23})$, $(G_{24})$, $(G_{31})$ et $(G_{32})$ des formules respectives (III), (IV), (V), (VI), (VII) et (VIII) telles que définies précédemment, un reste de dérivés saccharidiques sans liaison glycosidique établie entre un carbone anomérique et l'oxygène d'un groupement acétal, tels qu'ils sont définis dans l'ouvrage de référence: "Biochemistry, Daniel Voet/Judith G. Voet, p. 250, John Wyley & Sons, 1990.". Les structures oligomériques $(G_{21})_r$, $(G_{22})_s$, $(G_{23})_t$, $(G_{24})_u$, $(G_{31})_x$ et $(G_{32})_y$ peuvent se présenter sous toutes formes d'isoméries, qu'il s'agisse d'isomérie optique, d'isomérie géométrique ou d'isomérie de position ; elle peut aussi représenter un mélange d'isomères.

Dans les formules (III), (IV), (V), (VI), (VII) et (VIII) telles que définies ci-dessus, les groupes $R_{21}$, $R_{22}$, $R_{23}$, $R_{24}$, $R_{31}$ et $R_{32}$, sont liés respectivement aux restes $(G_{21})$, $(G_{22})$, $(G_{23})$, $(G_{24})$, $(G_{31})$ et $(G_{32})$ par leur carbone anomérique, de manière à former une fonction acétal.

Selon un aspect particulier de la présente invention la composition (EM) telle que définie ci-dessus, comprend pour 100% de sa masse,

1) - une proportion supérieure ou égal à 20% massique et inférieure ou égale à 70% dudit agent diluant (D) ou du mélanges des dits agents diluant (D), et

2) - Une proportion supérieure ou égale à 30% massique et inférieure ou égale à 80% massique de ladite composition (C) telle que définie précédemment ; et plus particulièrement la composition (EM) telle que définie ci-dessus, comprenant pour 100% de sa masse,

1) - une proportion supérieure ou égal à 20% massique et inférieure ou égale à 60% dudit agent diluant (D) ou du mélanges des dits agents diluant (D), et

**2) -** Une proportion supérieure ou égale à 40% massique et inférieure ou égale à 80% massique de ladite composition (C) telle que définie précédemment.

Selon un autre aspect particulier de la présente invention la composition (C) telle que définie ci-dessus, comprend pour 100% de sa masse :

**a) -** Une proportion supérieure à 0% et inférieure ou égale à 3% massique d'au moins un alcool de formule (II), telle que définie précédemment et
**b) -** Une proportion supérieure ou égale à 97% massique et inférieure à 100% massique de la composition ($C_1$) telle que définie précédemment ; et plus particulièrement la composition (C) telle que définie ci-dessus, comprend pour 100% de sa masse :

**a) -** Une proportion supérieure à 0% et inférieure ou égale à 2% massique d'au moins un alcool de formule (II), telle que définie précédemment et
**b) -** Une proportion supérieure ou égale à 98% massique et inférieure à 100% massique de la composition ($C_1$) telle que définie précédemment.

Selon un autre aspect particulier de la présente invention la composition ($C_1$) telle que définie ci-dessus, comprend pour 100% de sa masse :

**$b_1$) -** Une proportion supérieure ou égale à 65% massique et inférieure ou égale 90% massique de la composition ($C_2$) telle que définie précédemment et
**$b_2$) -** Une proportion supérieure ou égale à 10% massique et inférieure ou égale à 35% massique d'une composition ($C_3$) telle que définie précédemment ; et plus particulièrement la composition ($C_1$) telle que définie ci-dessus, comprend pour 100% de sa masse :

**$b_1$) -** Une proportion supérieure ou égale à 70% massique et inférieure ou égale 90% massique de la composition ($C_2$) telle que définie précédemment et
**$b_2$) -** Une proportion supérieure ou égale à 10% massique et inférieure ou égale à 30% massique d'une composition ($C_3$) telle que définie précédemment.

Selon un autre aspect particulier de la présente invention la composition ($C_2$) telle que définie ci-dessus, comprend pour 100% de sa masse :

**$b_{1i}$) -** Une proportion supérieure ou égale à 8% massique et inférieure ou égale à 18% massique de la composition ($C_{21}$) telle que définie précédemment,
**$b_{1ii}$) -** Une proportion supérieure ou égale à 10% massique et inférieure ou égale à 18% massique de la composition ($C_{22}$) telle que définie précédemment ;
**$b_{1iii}$) -** Une proportion supérieure ou égale à 28% massique et inférieure ou égale à 40% massique, de la composition ($C_{23}$) telle que définie précédemment ;
**$b_{1iv}$) -** Une proportion massique supérieure ou égale à 35% massique et inférieure ou égale à 50% massique de la composition ($C_{24}$) telle que défini précédemment,

étant entendu que la somme des proportions massiques des compositions ($C_{21}$), (C22), (C23), et (C24), est égale à 100%.
Selon un autre aspect particulier de la présente invention la composition ($C_3$) telle que définie ci-dessus, comprend pour 100% de sa masse :

**$b_{2i}$) -** Une proportion supérieure ou égale à 30% massique et inférieure ou égale à 45% massique de la composition ($C_{31}$) telle que définie précédemment ;
**$b_{2ii}$) -** Une proportion supérieure ou égale à 55% massique et inférieure ou égale à 70% massique de la composition ($C_{32}$) telle que définie précédemment.

étant entendu que la somme des proportions massiques des compositions ($C_{31}$) et (C22) est égale à 100%.
Selon un aspect particulier de la présente invention, la composition (EM) telle que définie précédemment est caractérisée en ce que, dans les formules (III), (IV), (V), (VI), (VII) et (VIII), $G_{21}$, $G_{22}$, $G_{23}$, $G_{24}$, $G_{31}$ et $G_{32}$, identiques ou différents, représentent indépendamment l'un de l'autre le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane et le tallose. Les dits

restes $G_{21}$, $G_{22}$, $G_{23}$, $G_{24}$, $G_{31}$ et $G_{32}$, identiques ou différents, sont plus particulièrement choisis parmi le glucose, le xylose et l'arabinose.

Selon un aspect plus particulier de la présente invention, la composition (EM) telle que définie précédemment est caractérisée en ce que dans les formules (III), (IV), (V) et (VI), lesdits restes d'un sucre réducteur $G_{21}$, $G_{22}$, $G_{23}$, $G_{24}$, représentent un même reste de sucre réducteur choisi parmi le reste du glucose, le reste du xylose et le reste de l'arabinose, et tout particulièrement représentent le reste du glucose, ou le reste du xylose.

Selon un autre aspect particulier de la présente invention, la composition (EM) telle que définie précédemment est caractérisée en ce que dans les formules (III), (IV), (V) et (VI), r, s, t et u respectivement, représentent indépendamment l'un de l'autre un nombre décimal supérieur ou égal à 1,05 et inférieur ou égal à 2,5 ; ce nombre décimal est dans ce cas souvent inférieur ou égal à 2,0, et par exemple supérieur ou égal à 1,25 et inférieur ou égal à 2,0.

Selon un autre aspect plus particulier de la présente invention, la composition (EM) telle que définie précédemment est caractérisée en ce que dans les formules (VII) et (VIII), lesdits restes d'un sucre réducteur $G_{31}$ et $G_{32}$ représentent un même reste de sucre réducteur choisi parmi le reste du glucose, le reste du xylose et le reste de l'arabinose, et tout particulièrement représentent le reste du glucose, ou le reste du xylose.

Selon un autre aspect plus particulier de la présente invention, la composition (EM) telle que définie précédemment est caractérisée en ce que dans les formules (VII) et (VIII), x et y respectivement, représentent un nombre décimal supérieur ou égal à 1,05 et inférieur ou égal à 2,5; ce nombre décimal est dans ce cas souvent inférieur ou égal à 2,0, et par exemple supérieur ou égal à 1,25 et inférieur ou égal à 2,0.

Selon un autre aspect particulier de la présente invention, la composition (EM) telle que définie précédemment, est caractérisée en ce que le ratio massique :

$$\Delta = \text{Masse de la composition } (C_2)/\text{Masse de la composition } (C_3),$$

est supérieur ou égal à 1 et inférieur ou égal à 10, plus particulièrement supérieur ou égal à 1,0 et inférieur ou égal à 8,0, tout particulièrement supérieur ou égal à 1,5 et inférieur ou égal à 7,0, par exemple supérieur ou égal à 2,0 et inférieur ou égal à 7,0.

Selon un autre aspect particulier de la présente invention, la composition (EM) telle que définie précédemment est caractérisée à l'une ou quelconque des revendications 1 à 10 caractérisée en ce que l'agent diluant est le 1,3-propanediol ou le glycérol.

L'invention a aussi pour objet la composition (EM) telle que définie précédemment, caractérisée en ce qu'elle comprend en outre

3) - Jusqu'à 7% massique d'eau, et plus particulièrement jusqu'à 5% massique d'eau, étant entendu que la somme des proportions massiques dudit agent diluant, de ladite composition (C) et de l'eau est égale à 100%.

L'invention a également pour objet un procédé de préparation de la composition (EM) telle que définie précédemment, comprenant les étapes successives suivantes :

- Une <u>étape A)</u> de réaction, dans les proportions souhaitées, d'un sucre réducteur de formule (IX) ou un mélange de sucres réducteurs de formule (IX) :

$$\text{HO-(G)-H} \qquad \text{(IX)}$$

dans laquelle G représente le reste d'un sucre réducteur, avec un excès molaire d'un mélange d'alcools de formule (II) :

$$C_mH_{2m+1}\text{-OH} \qquad \text{(II)},$$

telle que définie précédemment, pour former un mélange de composés de formule (III), (IV), (V), (VI), (VII) et (VIII) et un excès dudit mélange d'alcools de formule (II) ;

- Une <u>étape B)</u> d'élimination partielle de l'excès dudit mélange d'alcools de formule (II) pour former ladite composition (C) telle que définie précédemment ;
- une <u>étape C)</u> de mélange de ladite composition (C), avec au moins un agent diluant tel que défini précédemment, et si nécessaire ou si désiré,
- une <u>étape D</u> d'addition d'eau.

L'étape A) est généralement mise en œuvre dans un réacteur en présence d'un système catalytique acide, en maîtrisant le rapport stœchiométrique entre les deux réactants, et plus particulièrement en introduisant un excès molaire du mélange d'alcools de formule (II), sous agitation mécanique dans des conditions de température et de vide partiel prédéterminées, par exemple à une température comprise entre 70°C et 130°C et sous un vide partiel compris entre 300 mbar ($3.10^4$ Pa) et 20 mbar ($2.10^3$ Pa). Par système catalytique acide, on désigne les acides forts comme l'acide sulfurique, l'acide chlorhydrique, l'acide phosphorique, l'acide nitrique, l'acide hypophosphoreux, l'acide méthanesulfonique, l'acide para-toluène sulfonique, l'acide trifluorométhanesulfonique, ou les résines échangeuses d'ions.

Selon un aspect particulier du procédé objet de la présente invention, on introduit lors de l'étape A) de 4,0 à 10,0 équivalents molaires, plus particulièrement de 4,0 à 9,0 équivalents molaires, d'un mélange ($M_1$) comprenant pour 100% de sa propre masse :

- De 60% à 95% massique d'un mélange ($M_2$) comprenant pour 100% de sa propre masse :

  - de 8% à 16% massique de 1-dodécanol,

  - de 10% à 18% massique de 1-tétradécanol,

  - de 28% à 37% massique de 1-octanol,
  - de 29% à 54% massique de 1-décanol, et

- De 5% à 40% massique d'un mélange ($M_3$) comprenant pour 100% de sa propre masse :

  - de 30% à 50% massique de 1-hexadécanol, et
  - de 50% à 70% massique de 1-octadécanol,

et un équivalent molaire de glucose ou de xylose.

L'étape B) d'élimination partielle de l'excès dudit mélange d'alcools de formule (II) est généralement mise en œuvre selon des méthodes connues de l'homme du métier comme par exemple, la distillation, la distillation sur film à couche mince, la distillation sur film à couche mince à court trajet, la distillation moléculaire ou l'extraction par solvants, l'extraction par des fluides à l'état supercritique, comme par exemple le dioxyde de carbone supercritique ou le méthane supercritique, et plus particulièrement la distillation sur film à couche mince ou la distillation moléculaire ou l'extraction au dioxyde de carbone supercritique.

L'étape C) de mélange de ladite composition (C), obtenue à l'étape B), avec au moins un agent diluant est effectuée, soit en ligne soit dans un réacteur, sous agitation, dans des conditions de température et de pression spécifiques adaptées par l'homme du métier pour atteindre la composition (EM) objet de la présente invention, se présentant sous une forme liquide et homogène.

Le procédé tel que défini ci-dessus peut comprendre en outre des opérations de neutralisation, de filtration et de décoloration.

L'invention a également pour objet l'utilisation d'une composition (EM), telle que définie précédemment, comme agent émulsionnant pour la préparation d'une émulsion fluide de type huile-dans-eau à usage topique.

Dans le cadre de l'invention, on entend par « émulsion fluide » une émulsion dont l'écoulement au travers d'une coupe d'écoulement ISO 2431 de 6 millimètres commence moins de 5 secondes après l'enlèvement de l'obturateur (test selon la norme internationale ISO 2431). A titre d'émulsions fluides, on peut notamment citer les laits, en particulier les laits du type huile-dans-eau, à usage cosmétique ou hygiénique comme les laits démaquillants, les laits corporels, les laits solaires ou les laits autobronzants. A titre d'émulsions fluides vaporisables, on peut notamment citer les émulsions fluides pulvérisables comprenant un gaz propulseur cosmétiquement acceptable.

L'expression "à usage topique" utilisée dans la définition de l'émulsion de type huile-dans-eau, signifie que ladite émulsion de type huile-dans-eau est mise en œuvre par application sur la peau, les cheveux, le cuir chevelu ou les muqueuses, qu'il s'agisse d'une application directe dans le cas d'une préparation cosmétique, dermocosmétique, dermopharmaceutique ou pharmaceutique ou d'une application indirecte par exemple dans le cas d'un produit de soin corporel sous forme de lingette en textile ou en papier ou de produits sanitaires destinés à être en contact. avec la peau ou les muqueuses.

L'invention a également pour objet une émulsion de type huile-dans-eau (E), comprenant :

- de 50% à 80% massique d'au moins une phase aqueuse ($P_1$) cosmétiquement acceptable,
- de 0,5% à 15% massique, plus particulièrement de 0,5% à 10% massique, et encore plus particulièrement de 0,5% à 5% massique d'au moins une composition (EM) telle que définie précédemment, et
- de 5% à 49,5% massique, plus particulièrement de 10% à 49,5% massique, et encore plus particulièrement de 15% à 49,5% massique d'au moins une phase grasse ($P_2$),

étant entendu que la somme des proportions massiques de ladite phase aqueuse ($P_1$), de ladite composition (EM) et de ladite phase grasse ($P_2$) est égale à 100%.

Par "phase grasse (P2)", on désigne au sens de la présente invention, un corps gras ou un mélange de corps gras insoluble dans l'eau et/ou dans les mélanges d'eau et de solvants polaires. Une telle « phase grasse » peut comprendre des huiles et/ou des cires.

Parmi les éléments constitutifs de la phase grasse, on peut citer:

- Les huiles d'origine animale, telles que le squalène ou le squalane;
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de mais, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, l'huile de sésame, l'huile de reine des prés, l'huile de Macadamia kiwi, l'huile de bourrache, l'huile de pépins de cassis, l'huile de café, l'huile de pistache, l'huile de noyau de pêche, l'huile de pépin de framboise, l'huile de pépin de fraise, l'huile de melon, l'huile de pépin de myrtille, l'huile d'argan, l'extrait huileux de prune, l'huile de grenade, l'huile de papaye, l'huile de lait de coco, les huiles issues de fleurs ou de légumes;
- Les huiles végétales éthoxylées;
- Les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, le polydécène hydrogéné ou le polyisobutène hydrogéné, commercialisé en France par la société Ets B. Rossow et Cie sous le nom PARLEAM - POLYSYNLANE™, cité dans: Michel and Irene Ash; Thesaurus of Chemical Products, Chemical Publishing Co, Inc. 1986 Volume I, page 211 (ISBN 0 7131 36030);
- Les huiles de silicone comme les diméthylpolysiloxanes, les méthylphényl-polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.
- Des cires comme la cire d'abeille, la cire de carnauba, la cire de candelilla, la cire d'ouricoury, la cire du Japon, la cire de fibre de liège, la cire de canne à sucre, la cire de jojoba, la cire de fleurs de cassis, la cire de fleurs de narcisse, la cire de fleurs d'oranger, la cire d'orange, la cire de riz, les cires de paraffines, les cires de lignite, les cires microcristallines, la cire de lanoline; l'ozokérite; la cire de polyéthylène, les cires de silicone; les cires végétales; les alcools gras et les acides gras solides à température ambiante; les glycérides solides à température ambiante.

L'expression "cosmétiquement acceptable " utilisée dans la définition de la phase aqueuse ($P_1$) de l'émulsion huile-dans-eau et objet de la présente invention, qualifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Ladite phase aqueuse ($P_1$) cosmétiquement acceptable, peut contenir classiquement un ou plusieurs solvants organiques cosmétiquement acceptables, tels que les alcools polyhydriques comme le glycérol, le diglycérol, le triglycérol, les oligomères du glycérol, le xylitol, l'érythritol, le sorbitol, le méthyl-2-propanediol-1,3; les alcools polyhydriques alcoxylés; les glycols, comme le butylène glycol, l'hexylène glycol, le caprylyl glycol ou 1,2-octanediol, le pentylène glycol ou 1,2-pentanediol, le monopropylène glycol, le dipropylène glycol, l'isoprène glycol, le butyldiglycol, les polyéthylènes glycols dont le poids moléculaire est compris entre 200gmol$^{-1}$ et 8.000gmol$^{-1}$; ou les alcools hydrosolubles comme l'éthanol, l'isopropanol ou le butanol.

L'émulsion de type huile-dans-eau et telle que définie précédemment, est généralement préparée par la mise en œuvre d'un procédé comprenant :

- au moins une étape a) de mélange de la phase grasse ($P_2$) avec la composition émulsionnante (EM) telle que définie précédemment pour obtenir un mélange (M) ;
- au moins une étape b) d'émulsification du mélange (M) obtenu à l'issue de l'étape a) avec la phase aqueuse ($P_1$).

L'étape a) de mélange du procédé tel que défini ci-dessus, est effectuée à une température adaptée, en fonction du point de fusion des substances chimiques et/ou des compositions chimiques constituant la phase grasse (P$_2$), et par le moyen de tout dispositif de mélange connu de l'homme du métier, comme par exemple par le moyen d'un dispositif d'agitation équipé d'un mobile de type « ancre », à des vitesses d'agitations comprises entre 80 tours/minute et 1000 tours/minute, et comme par exemple par le moyen d'un dispositif d'agitation de type rotor-stator à des vitesses d'agitation comprises entre 1000 tours/minute et 8000 tours /minute.

Dans un tel procédé de préparation de l'étape b) d'émulsification du mélange (M) obtenu à l'issue de l'étape a) avec la phase aqueuse (P$_1$) peut être avantageusement mise en œuvre à une température inférieure ou égale à 80°C.

Cette étape b) du procédé de préparation de la formulation à usage topique (F1) objet de l'invention peut être réalisée par le moyen de tout dispositif de mélange connu de l'homme du métier, comme par exemple au le moyen d'un dispositif d'agitation de type rotor-stator à des vitesses d'agitation comprises entre 1000 tours/minute et 8000 tours /minute.

L'émulsion de type huile-dans-eau et telle que définie précédemment, peut également être préparée par la mise en œuvre d'un procédé comprenant :

- au moins une étape a') de mélange de la phase aqueuse (P$_1$) avec la composition émulsionnante (EM) telle que définie précédemment pour obtenir un mélange (M') ;
- au moins une étape b') d'émulsification du mélange (M') obtenu à l'issue de l'étape a') avec la phase grasse (P$_2$).

De façon générale l'émulsion de type huile-dans-eau et telle que définie précédemment, comporte également des excipients et ou des principes actifs habituellement mis en œuvre dans le domaine des formulations à usage topique, en particulier cosmétiques, dermocosmétiques, pharmaceutiques ou dermo-pharmaceutiques, comme les tensioactifs épaississants et/ou gélifiants, les stabilisants, les composés filmogènes, les agents hydrotropes, les agents plastifiants, les agents émulsionnants et co-émulsionnants, les agents opacifiants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture.

L'émulsion de type huile-dans-eau et telle que définie précédemment, peut être conditionnée dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

L'émulsion de type huile-dans-eau et telle que définie précédemment peut comprendre un ou plusieurs adjuvants tels que :

- Des agents épaississants ou gélifiants, par exemple es polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- méthyl)acrylamido méthane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymères linéaire, branché ou réticulé d'au moins un monomère possédant une fonction acide fort, libre, partiellement salifiée ou totalement salifiée, avec au moins un monomère neutre, et au moins un monomère de formule (XI):

$$CH_2=C(R'_3)-C(=O)-[CH_2-CH_2-O]_n-R'_4 \qquad (XI)$$

dans laquelle R'$_3$ représente un atome d'hydrogène ou un radical méthyle, R'$_4$ représente un radical alkyle linéaire ou ramifié comportant de huit à trente atomes de carbone et n représente un nombre supérieur ou égal à un et inférieur ou égal à cinquante; Les polymères de type polyélectrolytes, linéaires ou branchés ou réticulés que l'on peut associer à l'émulsion huile-dans-eau objet de la présente invention, peuvent se présenter sous la forme d'une solution, d'une suspension aqueuse, d'une émulsion eau-dans-huile, d'une émulsion huile-dans-eau, d'une poudre, par exemple les produits commercialisés sous les appellations SIMULGEL™EG, SIMULGEL™EPG, SEPIGEL™305, SIMULGEL™600, SIMULGEL™NS, SIMULGEL™INS100, SIMULGEL™FL, SIMULGEL™A, SIMULGEL™SMS88, SEPINOV™EMT10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC2, ARISTOFLEX™HMB, COSMEDIA™SP, FLOCARE™ET25, FLOCARE™ET75, FLOCARE™ET26, FLOCARE™ET30, FLOCARE™ET58, FLOCARE™PSD30, VISCOLAM™AT64, VISCOLAM™AT100; les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS=1/5), de la gomme de caroube (DS=1/4), de la gomme de tara (DS=1/3), de la gomme de guar (DS=1/2), de la gomme de fenugrec (DS=1); les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes; la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes;

- Des composés filmogènes,
- Des agents hydrotropes,
- Des agents plastifiants,
- Des agents opacifiants et/ou nacrants, tels que les palmitate, stéarate ou les hydroxy-stéarate de sodium ou de magnésium, les monostéarates ou distéarate d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.
- Des agents de texture tels que la lauroyl lysine commercialisée sous l'appellation AMINOHOPE™LL par la société AJINOMOTO, l'octenyl starch succinate commercialise sous l'appellation DRYFLO™ par la société NATIONAL STARCH, le myristyl polyglucoside commercialisé par SEPPIC sous l'appellation MONTANOV™ 14, les fibres de cellulose, les fibres de coton, les fibres de chitosane, le talc, la sericite, le mica;
- Des agents surgraissants,
- Des agents séquestrants,
- Des agents chélatants,
- Des agents tensioactifs non-ioniques tels que les dérivés éthoxylés d'alcools gras comportant de 8 à 12 atomes de carbone, les dérivés éthoxylés d'acides gras comportant de 8 à 12 atomes de carbone; les dérivés éthoxylés d'esters gras comportant de 8 à 12 atomes de carbone; les dérivés éthoxylés de monoglycérides comportant de 8 à 12 atomes de carbone; les alkyl polyglycosides de formule (XII):

$$R_2\text{-O-}(S')_z\text{-H} \qquad (XII)$$

dans laquelle z représente un nombre décimal compris entre 1 et 5, S' représente le reste d'un sucre réducteur et R$_2$ représente un radical alkyle linéaire ou ramifié, saturé ou insaturé, ayant de 5 à 16 atomes de carbone, de préférence de 8 à 14 atomes de carbone, ou un mélange de composés de formule (II), par exemple le caprylyl capryl glucosides, commercialisé notamment sous le nom de marque ORAMIX™CG 110, le décylglucoside, commercialisé notamment sous le nom de marque ORAMIX™NS 10;

- Des agents antioxydants, tels que l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S (nom INCI: Tetrasodium Glutamate Diace-tate);
- Des parfums,
- Des agents conservateurs,
- Des agents conditionneurs,
- Des principes actifs destinés à apporter une action traitante vis à vis de la peau ou des cheveux, tels que les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés); les composés montrant une action apaisante notamment le SEPICALM™ S, l'allantoïne et le bisabolol; les agents anti-inflammatoires; les composés montrant une action hydratante comme l'urée, les hydroxyurées, le glycérolglucoside, le diglycérolglucoside, les polyglycérylglucosides; les extraits végétaux riches en polyphénols comme les extraits de raisin, les extraits de pin, les extraits de vin, les extraits d'olives; les composés montrant une action amincissante ou lipolytique comme la caféine ou ses dérivés, l'ADIPOSLIM™, l'ADIPOLESS™, la fucoxanthine; les protéines N-acylés; les peptides N-acylés comme le MATRIXIL™; les acides aminés N-acylés; les hydrolysâts partiels de protéines N-acylés; les acides aminés; les peptides; les hydrolysâts totaux de protéines; les extraits de soja, par exemple la Raffermine™; les extraits de blé par exemple la TENSINE™ ou la GLIADINE™; les extraits végétaux, tels que les extraits végétaux riches en tanins, les extraits végétaux riches en isoflavones ou les extraits végétaux riches en terpènes; les extraits d'algues d'eau douce ou marines; les extraits de plantes marines; les extraits marins en général comme les coraux; les cires essentielles; les extraits bactériens; les céramides; les phospholipides; les composés montrant une action antimicrobienne ou une action purifiante, comme le LIPACIDE™ C8G, le LIPACIDE™ UG, le SEPICONTROL™ A5; l'OCTOPIROX™ ou le SENSIVA™ SC50; les composés montrant une propriété énergisante ou stimulante comme le PHYSIOGENYL™, le panthénol et ses dérivés comme le SEPICAP™ MP; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™; le SURVICODE™; les actifs anti-photo vieillissement; les actifs protecteurs de l'intégrité de la jonction dermo-épidermique; les actifs augmentant la synthèse des composants de la matrice extracellulaire comme le collagène, les élastines, les glycosaminoglycanes; les actifs agissant favorablement sur la communication cellulaire chimique comme les cytokines ou physiques comme les intégrines; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés); les actifs améliorant la microcirculation cutanée, par exemple les veinotoniques; les actifs drainants; les actifs à visée décongestionnante comme les extraits de ginko biloba, de lierre, de marron d'inde, de bambou, de ruscus, de petit houx, de centalla asiatica, de fucus, de romarin, de saule; les agents de bronzage ou de brunissement de la peau, comme par exemple la dihydroxyacétone (DHA), l'érythrulose, l'aldéhyde mésotartrique, le glutaraldéhyde, le glycéraldéhyde, l'alloxane, la ninhydrine, les extraits végétaux comme par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683;; les agents connus pour leur action de facilitation et/ou d'accélération du bronzage et/ou du brunissement de la peau humaine, et/ou pour leur action de coloration de la peau humaine, comme par exemple les caraténoïdes (et plus particulièrement le beta carotène et le gamma carotène), le produit commercialisé sous le nom de marque « Carrot oil » (Nom INCI: Daucus Carota, helianthus annuus Sunflower oil) par la société Provital, qui contient des caroténoïdes, de la vitamine E et de la vitamine K; la tyrosine et/ou ses dérivés, connus pour leur effet sur l'accélération du bronzage de la peau humaine en association avec une exposition aux rayonnements ultra-violets, comme par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI: Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI: butylene glycol and Acetyl Tyrosine and hydrolyzed vegetable protein and Adenosine triphosphate) par la société UNIPEX, le produit commercialisé sous le nom de marque « Try-Excell™ » (nom INCI: Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™ » (nom INCI: hydrolyzed wheat protein and acetyl tyrosine and copper gluconate) par la société Alban Muller, le produit commercialisé sous le nom de marque Tyrostan™ (nom INCI: potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze™ (nom INCI: Dihydroxyacetone and acetyl tyrosine and copper gluconate) commercialisé par la société Alban Muller, le produit commercialisé sous le nom de marque Tyrosilane (nom INCI: méthylsilanol and acétyl tyrosine) par la société Exymol; les peptides connus pour leur effet d'activation de la mélanogénèse comme par exemple le produit commercialisé sous le nom de marque Bronzing SF Peptide powder (nom INCI: Dextran and Octapeptide-5) par la société Infinitec Activos, le produit commercialisé sous le nom de marque Melitane (nom INCI: Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI: Butylene glycol, Palmitoyl Tripeptide-40) par la société LIPOTEC, les sucres et les dérivés de sucres comme par exemple le produit commercialisé sous le nom de marque Tanositol™ (nom INCI: inositol) par la société Provital, le produit commercialisé sous le nom de marque Thalitan™ (ou Phycosaccharide™ AG) par la société CODIF international (nom INCI: Aqua and hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI: Maltodextrin, Mucuna Pruriens Seed extract) par la société Alban Muller, les composés riches en flavonoïdes comme par exemple le produit commercialisé sous le nom de marque « Biotanning » (nom INCI: Hydrolyzed citrus Aurantium dulcis fruit extract) par la société Silab et connu pour être riche en flavonoides de citron (de type hespéridines);

- Des charges minérales ou des pigments, tels que le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.
- Des particules procurant un effet visuel ou destinées à l'encapsulation d'actifs,
- Des particules exfoliantes,
- Des azurants optiques,
- Des agents répulsifs des insectes.

Parmi les agents de protection contre les rayonnements ultra-violets du soleil que peut comprendre l'émulsion huile-dans-eau objet de la présente invention, on peut citer les pigments, les filtres organiques solaires et les filtres inorganiques solaires.

Comme pigments utilisés comme agent de protection contre les rayonnements ultra-violets du soleil que peut comprendre l'émulsion huile-dans-eau objet de la présente invention, il y a par exemple le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Comme filtres organiques solaires utilisés comme agent de protection contre les rayonnements ultra-violets du soleil que peut comprendre l'émulsion huile-dans-eau objet de la présente invention, il y a par exemple :

- Ceux de la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Ceux de la famille des dérivés de l'acide anthranilique comme l'homomenthyl-N-acétyl anthranilate;
- Ceux de la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle;
- Ceux de la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-□α-cyano-β-phényle, le cinnamate de diparaméthoxy mono-2-éthylhexanoyl de glycéryle;
- Ceux de la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 2-éthylhexyl 4'-phényl benzophénone-2-carboxylate, la 2-hydroxy 4-n-octyloxy benzophénone, la 4-hydroxy 3-carboxy benzophénone ; le 3-(4'-méthylbenzylidène) d,l-camphre, le 3-(benzylidène)-d,l-camphre, le benzalkonium méthosulfate camphre ; l'acide urocanique, l'urocanate d'éthyle ;
- Ceux de la famille des dérivés de l'acide sulfonique comme l'acide 2-phényl benzimidazole-5-sulfonique et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy) 1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthyl benzoxazole, le 2,2'-hydroxy-5-méthylphényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbornylidène)-3-pentan-2-one ; le 2-(4-diethylamino 2-hydroxy benzoyl) benzoic acid hexyl ester, le 2,4-bis{[4-(2-ethylhexyloxy)-2-hydroxy] phenyl}-6-(4-methoxy phenyl) 1,3,5-triazine, le 2,4,6-tris[4-(2-ethylhexyloxycarbonyl) anilino]-1,3,5-triazine, le 2-ethylhexyl dimethoxybenzylidene dioxoimidazolidine propionate, la famille des dérivés du diphénylacrylate comme le 2-éthylhexyl-2-cyano-3,3-diphényl-2-propènoate, l'éthyl-2-cyano-3,3-diphényl-2-propènoate;
- Ceux de la famille des polysiloxanes comme le malonate de benzylidène siloxane.

Comme filtres inorganiques solaires utilisés comme agent de protection contre les rayonnements ultra-violets du soleil que peut comprendre l'émulsion huile-dans-eau objet de la présente invention, il y a par exemple: les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Les exemples suivants illustrent l'invention sans toutefois la limiter.

**Exemple 1: Préparation d'une composition émulsionnante (EM$_1$) selon l'invention**

On prépare 72,5kg (soit 8,5 équivalents molaires) d'un mélange (M$_1$) de 1-octanol, 1-décanol, 1-dodécanol, 1-tétradécanol, 1-hexadécanol et 1-octadécanol en introduisant sous agitation et à 90°C successivement chacun des alcools gras précités, dans des proportions telles que la répartition massique de chacun des alcools pour 100% massique dudit mélange (M$_1$) est la suivante :

- 1-octanol = 22,1% massique ;
- 1-décanol = 27,9% massique ;
- 1-dodécanol = 11,1% massique ;
- 1-tétradécanol = 13,1% massique ;
- 1-hexadécanol = 10,7% massique ; et
- 1-octadécanol = 15,1% massique.

Le mélange (M$_1$) est homogénéisé à 90°C pendant trente minutes, puis additionné de 8,7kg (soit un équivalent molaire) de glucose, puis de 80g d'acide sulfurique à 98%.

Le milieu réactionnel est mis sous un vide partiel de 90mbar à 45mbar, et maintenu à une température de 100°C à 105°C pendant cinq heures avec évacuation de l'eau formée au moyen d'un montage de distillation.

Le milieu réactionnel est ensuite refroidi à 80°C et neutralisé par ajout de 76g de soude à 40%, Le produit est ensuite filtré sur plaques de façon à éliminer le glucose non réagi, et on obtient 71,8kg d'un milieu comprenant les espèces glucosidiques formées ainsi que les alcools gras en excès stœchiométrique n'ayant pas réagi.

Les alcools résiduels présents dans le mélange ainsi obtenu sont ensuite éliminés par passage dans un évaporateur sur film à couche mince.

36,6kg du milieu réactionnel ainsi neutralisé est alors introduit dans un évaporateur à film à couche mince, sous une pression réduite de 3 à 5 mbar avec une température de paroi de 240°C, de façon à distiller la majeure partie des alcools résiduels et à obtenir 4,1kg d'un concentré, auquel 2,73kg de glycérol sont ajoutés progressivement sous agitation, de façon à obtenir 6,83kg de la composition (EM$_1$).

**Exemple 2: Préparation d'une composition émulsionnante (EM$_2$) selon l'invention**

Le mode opératoire de l'exemple 1 décrit ci-dessus est reproduit pour préparer 8,5 équivalents molaires d'un mélange (M$_2$) comprenant pour 100% massique, 24,7% massique de 1-octanol, 32,0% massique de 1-décanol; 10,9% massique de 1-dodécanol, 13,4% massique de 1-tétradécanol, 7,6% massique de 1-hexadécanol et 11,4% massique de 1-octadécanol. Selon ce même mode opératoire, on prépare aussi la composition émulsionnante (EM$_2$) comprenant pour 100% massique, 30% massique de glycérol.

**Exemple 3: Préparation d'une composition émulsionnante (EM$_3$) selon l'invention**

Le mode opératoire de l'exemple 1 décrit ci-dessus est reproduit pour préparer 8,5 équivalents molaires d'un mélange (M$_3$) comprenant pour 100% massique, 30,2% massique de 1-octanol, 38,4% massique de 1-décanol; 7,4% massique de 1-dodécanol, 8,9% massique de 1-tétradécanol, 5,1% massique de 1-hexadécanol et 10,0% massique de 1-octadécanol. Selon ce même mode opératoire, on prépare aussi la composition émulsionnante (EM$_3$) comprenant pour 100% massique, 50% massique de glycérol.

**Exemple 4: Préparation d'une composition émulsionnante (EM$_5$) selon l'invention**

Le mode opératoire de l'exemple 2 décrit ci-dessus est reproduit pour préparer la composition émulsionnante (EM$_5$) comprenant pour 100% massique, 50% massique de 1,3-propanediol.

**Exemple 5: Préparation d'une composition émulsionnante (EM$_6$) selon l'invention**

Le mode opératoire de l'exemple 2 décrit ci-dessus est reproduit pour préparer la composition émulsionnante

(EM$_6$) comprenant pour 100% massique, 50% massique de 2-méthyl 2,4-pentanediol.

**Exemple 6: Préparation d'une composition émulsionnante (EM$_7$) selon l'invention**

Le mode opératoire de l'exemple 2 décrit ci-dessus est reproduit pour préparer la composition émulsionnante (EM$_6$) comprenant pour 100% massique, 50% massique de 1,3-butanediol.

Dans le tableau 1 sont recensées des caractéristiques analytiques des compositions émulsionnante (EM$_1$), (EM$_2$), (EM$_3$), (EM$_5$), (EM$_6$) et (EM$_7$) selon l'invention.

**Tableau 1**

|  | (EM$_1$) | (EM$_2$) | (EM$_3$) |
|---|---|---|---|
| Aspect (visuel) | Liquide | Liquide | Liquide |
| Glycérol[1] | 40% | 30% | 49,3% |
| Teneur totale [2] en Alcools gras C$_8$ à C$_{18}$[1] | 4,4% | 2,1% | 0,7% |
| - Teneur en APG$_{C8}$[1]<br>- Degré polymérisation APGcs [1'] | 12,3%<br>1,15 | 16,8%<br>1,15 | 15,1%<br>1,15 |
| - Teneur en APG$_{C10}$[1]<br>- Degré polymérisation APG$_{C10}$ [1'] | 15,5%<br>1,15 | 21,7%<br>1,15 | 19,2%<br>1,15 |
| - Teneur en - APG$_{C12}$[1]<br>- Degré polymérisation APG$_{C12}$ [1'] | 6,2%<br>1,15 | 7,4%<br>1,15 | 3,7%<br>1,15 |
| - Teneur en APG$_{C14}$[1]<br>- Degré polymérisation APG$_{C14}$ [1'] | 7,3%<br>1,15 | 9,1%<br>1,15 | 4,5%<br>1,15 |
| - Teneur en APG$_{C16}$[1]<br>- Degré polymérisation APG$_{C16}$ [1'] | 5,9%<br>1,13 | 5,2%<br>1,13 | 2,5%<br>1,13 |
| - Teneur en APG$_{C18}$[1]<br>- Degré polymérisation APG$_{C18}$ [1'] | 8,4%<br>1,13 | 7,7%<br>1,13 | 5,0%<br>1,13 |
| Ratio Δ[3] | 2,9 | 4,3 | 5,7 |

**Tableau 1 (suite)**

|  | (EM$_5$) | (EM$_5$) | (EM$_6$) |
|---|---|---|---|
| Aspect (visuel) | Liquide | Liquide | Liquide |
| 1,3 propanediol[1] | 50% | 0% | 0% |
| 2-méthyl 2,4-pentanediol[1] | 0% | 50% | 0% |
| 1,3 butanediol[1] | 0% | 0% | 50% |
| Teneur totale [2] en Alcools gras C$_8$ à C$_{18}$[1] | 0,9% | 0,9% | 0,9% |
| - Teneur en APG$_{C8}$[1] - Degré polymérisation APGcs [1'] | 12,2% 1,15 | 12,2% 1,15 | 12,2% 1,15 |
| - Teneur en APG$_{C10}$[1]<br>- Degré polymérisation APG$_{C10}$ [1'] | 15,7%<br>1,15 | 15,7%<br>1,15 | 15,7%<br>1,15 |
| - Teneur en - APG$_{C12}$[1]<br>- Degré polymérisation APG$_{C12}$ [1'] | 5,3%<br>1,15 | 5,3%<br>1,15 | 5,3%<br>1,15 |
| - Teneur en APG$_{C14}$[1]<br>- Degré polymérisation APG$_{C14}$ [1'] | 6,7%<br>1,15 | 6,7%<br>1,15 | 6,7%<br>1,15 |
| - Teneur en APG$_{C16}$[1]<br>- Degré polymérisation APG$_{C16}$ [1'] | 3,7%<br>1,13 | 3,7%<br>1,13 | 3,7%<br>1,13 |
| - Teneur en APG$_{C18}$[1] | 5,6% | 5,6% | 5,6% |

(suite)

|  | (EM$_5$) | (EM$_5$) | (EM$_6$) |
|---|---|---|---|
| - Degré polymérisation APG$_{C18}$ [(1')] | 1,13 | 1,13 | 1,13 |
| Ratio $\Delta$[(3)] | 4,3 | 4,3 | 4,3 |
| (1) Quantification par mise en œuvre méthode chromatographie en phase gazeuse.<br>(1') Détermination par mise en œuvre méthode chromatographie en phase gazeuse (méthode de Flory).<br>(2) Teneur totale en alcools gras C$_8$ à C$_{18}$ = teneur en 1-octanol + teneur en 1-décanol + teneur en 1-dodécanol + teneur en 1-tétradécanol + teneur en 1-hexadécanol + teneur en 1-octadécanol ; la teneur en chaque alcool étant déterminée par une méthode de chromatographie en phase gazeuse.<br>(3) Ratio $\Delta$ = [(Teneur en APG$_{C8}$)+(Teneur en APG$_{C10}$) + (Teneur en APG$_{C12}$) + (Teneur en APG$_{C14}$)] / [(Teneur en APG$_{C16}$) + (Teneur en APG$_{C18}$)]. | | | |

Dans le tableau 2 sont recensées les répartitions massiques relatives des espèces alkylpolyglucosidiques (APG) selon les différentes chaines alkyles dans les compositions (EM$_1$), (EM$_2$), (EM$_3$), (EM$_5$), (EM$_6$) et (EM$_7$) selon l'invention.

**Tableau 2**

|  | (EM$_1$) | (EM$_2$) | (EM$_3$) | (EM$_5$) | (EM$_6$) | (EM$_7$) |
|---|---|---|---|---|---|---|
|  | Répartition relative (pour 100% massique) des APGcs à APG$_{C14}$ | | | | | |
| APG$_{C8}$ | 29,8% | 30,5% | 35,5% | 30,5% | 30,5% | 30,5% |
| APG$_{C10}$ | 37,5% | 39,5 % | 45,2% | 39,5 % | 39,5 % | 39,5 % |
| APG$_{C12}$ | 15,0% | 13,5% | 8,7% | 13,5% | 13,5% | 13,5% |
| APG$_{C14}$ | 17,7% | 16,5% | 10,6% | 16,5% | 16,5% | 16,5% |
|  | Répartition relative (pour 100% massique) des APG$_{C16}$ à APG$_{C18}$ | | | | | |
| APG$_{C16}$ | 41,3% | 40,3% | 33,3% | 40,3% | 40,3% | 40,3% |
| APG$_{C18}$ | 58,7% | 59,7% | 66,7% | 59,7% | 59,7% | 59,7% |

**Exemple 4: Préparation d'émulsions huile-dans-eau mettant en œuvre les compositions émulsionnantes (EM$_3$), (EM$_5$) (EM$_6$) et (EM$_7$) selon l'invention et la composition (EM$_4$) selon l'état de la technique.**

On prépare une série d'émulsions huile-dans-eau (E$_1$) à (E$_8$) selon l'invention, comprenant la composition (EM$_3$), dont les compositions et les caractérisations sont indiquées dans le tableau 3 ci-dessous, en mettant en œuvre dans le procédé suivant :

**Etape a)** : La composition émulsionnante à tester est ajoutée sur une huile préalablement introduite dans un réacteur porté à une température de 45°C, et le mélange obtenu est homogénéisé pendant trente minutes au moyen d'un agitateur à ancre.

**Etape b)** : La gomme xanthane est ajoutée au mélange préparé à l'issue de l'étape a) et le tout est homogénéisée à une température de 45°C, pendant quinze minutes au moyen d'un agitateur à ancre.

**Etape c)** : La phase aqueuse est ensuite introduite sur le mélange préparé à l'issue de l'étape b), maintenu à une température de 45°C. Le tout est homogénéisé pendant quinze minutes au moyent d'un agitateur à ancre.

**Etape d)** : Le mélange obtenu lors de l'étape c) est agité énergiquement au moyen d'un émulseur de type rotor-stator, commercialisé par la société SILVERSON, pendant quatre minutes. Le mélange résultant est ensuite refroidi à température ambiante.

**Etape e)** : L'agent conservateur (Geogard™ 221), et le cas échéant le chlorure de sodium, sont ajoutés au mélange obtenu lors de l'étape d). Le tout est homogénéisé pendant quinze minutes au moyen d'un agitateur à ancre.

La moitié de la quantité de chaque émulsion (E$_1$) à (E$_8$) ainsi préparée est conservée à une température de 20°C pendant trois mois. L'autre moitié est conservée à une température de 45°C pendant trois mois. A l'issue de cette période de 3 mois, l'aspect de chaque émulsion préparée est observé. Les compositions (en pourcentages massiques) et la caractérisation des émulsions E$_1$ à E$_8$, sont recensées dans le tableau 3 suivant :

**Tableau 3**

| | Emulsions | | | | | | | |
|---|---|---|---|---|---|---|---|---|
| | $E_1$ | $E_2$ | $E_3$ | $E_4$ | $E_5$ | $E_6$ | $E_7$ | $E_8$ |
| Composition ($EM_3$) | 3% | 3% | 3% | 3% | 3% | 3% | 3% | 3% |
| Huile d'amande douce | 20% | 20% | 0% | 0% | 0% | 0% | 0% | 0% |
| Huile de tournesol | 0% | 0% | 20% | 20% | 0% | 0% | 0% | 0% |
| Phytosqualane | 0% | 0% | 0% | 0% | 20% | 20% | 0% | 0% |
| Lanol™ 2681 [7] | 0% | 0% | 0% | 0% | 0% | 0% | 20% | 20% |
| Keltrol™ CG-T[5] | 0,5% | 0,5% | 0,5% | 0,5% | 0,5% | 0,5% | 0,5% | 0,5% |
| Geogard™ 221[6] | 0,6% | 0,6% | 0,6% | 0,6% | 0,6% | 0,6% | 0,6% | 0,6% |
| Chlorure de sodium | 0% | 1,0% | 0% | 1,0% | 0% | 1,0% | 0% | 1,0% |
| Eau | Qsp 100% | Qsp 100% | Qsp 100% | Qsp 100% | Qsp 100% | Qsp 100% | Qsp 100% | Qsp 100% |
| | Aspect après trois mois | | | | | | | |
| à 20°C | H[a] | H | H | H | H | H | H | H |
| à 45°C | H | H | H | H | H | H | H | H |
| | Viscosité après 7 jours à 20°C(BKF LVT Mobile 3, Vitesse 6) | | | | | | | |
| En mPas | 3.500 | 4.600 | 3.500 | 4.500 | 3.300 | 5.800 | 3.800 | 5.700 |
| (5) : Keltrol™ CG-T est une gomme xanthane utilisée comme agent épaississant et/ou gélifiant<br>(6) : Geogard™ 221 (nom INCI Dehydroacetic Acid (and) Benzyl Alcohol) est une composition utilisée comme agent conservateur.<br>(7) Lanol™ 2681 (nom INCI Coco-caprylate/caprate) est une phase grasse se présentant sous forme liquide à 20°C<br>(a) : H : aspect homogène | | | | | | | | |

On prépare une autre série d'émulsions huile-dans-eau ($E_9$) à ($E_{17}$) selon l'invention, comprenant les compositions ($EM_5$), ($EM_6$) et ($EM_7$) dont les compositions et les caractérisations sont indiquées dans le tableau 4 ci-dessous, en mettant en œuvre les étapes a) à e) du procédé de préparation des émulsions décrits ci-dessus.

La moitié de la quantité de chaque émulsion ($E_9$) à ($E_{17}$) ainsi préparée est conservée à une température de 20°C pendant trois mois. L'autre moitié est conservée à une température de 45°C pendant trois mois. A l'issue de cette période de 3 mois, l'aspect de chaque émulsion préparée est observé. Les compositions (en pourcentages massiques) et la caractérisation des émulsions $E_9$ à $E_{17}$, sont recensées dans le tableau 4 suivant :

**Tableau 4**

| | Emulsions | | | | | | | | |
|---|---|---|---|---|---|---|---|---|---|
| | $E_9$ | $E_{10}$ | $E_{11}$ | $E_{12}$ | $E_{13}$ | $E_{14}$ | $E_{15}$ | $E_{16}$ | $E_{17}$ |
| Composition ($EM_5$) | 3% | 3% | 3% | 3% | 0% | 0% | 0% | 0% | 0% |
| Composition ($EM_6$) | 0% | 0% | 0% | 0% | 3% | 3% | 3% | 0% | 0% |
| Composition ($EM_7$) | 0% | 0% | 0% | 0% | 0% | 0% | 0% | 3% | 3% |
| Huile d'amande douce | 20% | 0% | 0% | 0% | 20% | 0% | 0% | 20% | 0% |

(suite)

| | Emulsions | | | | | | | | |
|---|---|---|---|---|---|---|---|---|---|
| | $E_9$ | $E_{10}$ | $E_{11}$ | $E_{12}$ | $E_{13}$ | $E_{14}$ | $E_{15}$ | $E_{16}$ | $E_{17}$ |
| Huile de tournesol | 0% | 20% | 0% | 0% | 0% | 20% | 0% | 0% | 20% |
| Phytosqualane | 0% | 0% | 20% | 0% | 0% | 0% | 0% | 0% | 0% |
| Lanol™ 2681[7] | 0% | 0% | 0% | 20% | 0% | 0% | 20% | 0% | 0% |
| Keltrol™ CG-T[5] | 0,5% | 0,5% | 0,5% | 0,5% | 0,5% | 0,5% | 0,5% | 0,5% | 0,5% |
| Geogard™ 221[6] | 0,6% | 0,6% | 0,6% | 0,6% | 0,6% | 0,6% | 0,6% | 0,6% | 0,6% |
| Chlorure de sodium | 0% | 0% | 0% | 0% | 0% | 0% | 0% | 0% | 0% |
| Eau | Qsp 100% | Qsp 100% | Qsp 100% | Qsp 100% | Qsp 100% | Qsp 100% | Qsp 100% | Qsp 100% | Qsp 100% |
| | Aspect après trois mois | | | | | | | | |
| à 20°C | H[a] | H | H | H | H | H | H | H | H |
| à 45°C | H | H | H | H | H | H | H | H | H |
| | Viscosité après 7 jours à 20°C(BKF LVT Mobile 3, Vitesse 6) | | | | | | | | |
| En mPas | 3.060 | 4.240 | 2.690 | 3.120 | 3.060 | 4.240 | 3.140 | 3.040 | 3.260 |

Les résultats consignés dans le tableau 3 font apparaître que la composition ($EM_3$) selon l'invention permet d'obtenir des émulsions huile-dans-eau $E_1$ à $E_8$, stables au stockage, à 20°C et à 45°C, en absence ou en présence de 1% de chlorure de sodium.

Les résultats consignés dans le tableau 4 font également apparaître que les compositions ($EM_5$), ($EM_6$) et ($EM_7$) selon l'invention permettent d'obtenir des émulsions huile-dans-eau $E_9$ à $E_{17}$, stables au stockage, à 20°C et à 45°C.

**Formulations illustratives**

Dans les formulations suivantes, les pourcentages sont exprimés en pourcentage massique pour 100% de la masse de la formulation.

**Exemple 5 : Crème de soin**

| | | |
|---|---|---|
| Cyclométhicone : | | 10% |
| SIMULGEL™ EG : | | 0,8% |
| Composition ($EM_3$) : | | 2% |
| Alcool stéarylique : | | 1% |
| Alcool stéarique : | | 0,5% |
| Conservateur : | | 0,65% |
| Lysine : | | 0,025% |
| EDTA (sel disodique) : | | 0,05% |
| Gomme de xanthane : | | 0,2% |
| Glycérine : | | 3% |
| Eau : | q.s.p. | 100% |

**Exemple** 6 : **Lait solaire**

FORMULE

| A | Composition (EM$_1$) : | | 3,0% |
|---|---|---|---|
| | Huile de sésame : | | 5,0% |
| | PARSOL™ MCX : | | 5,0% |
| | Carraghénane λ : | | 0,10% |
| B | Eau : | q.s.p. | 100% |
| C | SIMULGEL™ NS : | | 0,80% |
| D | Parfum : | | q.s. |
| | Conservateur : | | q.s |

MODE OPERATOIRE

Emulsionner B dans A à 60°C puis ajouter C vers 60°C, puis D vers 30°C et ajuster le pH si nécessaire.

**Exemple 7** : **Lait corporel**

| Composition (EM$_2$) : | | 3,5% |
|---|---|---|
| LANOL™ 37T : | | 8,0% |
| SOLAGUM™ L : | | 0,05% |
| Eau : | q.s.p. | 100% |
| Benzophénone-3 : | | 2,0% |
| Diméthicone 350cPs : | | 0,05% |
| SIMULGEL™ NS : | | 2,5% |
| Conservateur : | | 0,2% |
| Parfum : | | 0,4% |

**Exemple 8 : Emulsion démaquillante à l'huile d'amandes douces**

| Composition (EM3) : | | 5% |
|---|---|---|
| Huile d'amandes douces : | | 5% |
| Eau : | q.s.p. | 100% |
| SIMULGEL™ INS 100 : | | 0,3% |
| Glycérine : | | 5% |
| Conservateur : | | 0,2% |
| Parfum : | | 0,3% |

**Exemple 9 : Crème hydratante pour peaux grasses**

| Composition (EM$_1$) : | | 5% |
|---|---|---|
| Cétylstéaryloctanoate : | | 8% |
| Palmitate d'octyle : | | 2% |
| Eau : | q.s.p. | 100% |
| SIMULGEL™ NS : | | 2,6% |

(suite)

| | |
|---|---|
| MICROPEARL™ M100 : | 3,0% |
| Mucopolysaccharides : | 5% |
| SEPICIDE™ HB : | 0,8% |
| Parfum : | 0,3% |

**Exemple 10 : Lait démaquillant**

| | | |
|---|---|---|
| Composition (EM$_3$) : | | 3% |
| PRIMOL™ 352 : | | 8,0% |
| Huile d'amandes douces : | | 2% |
| Eau : | q.s.p. | 100% |
| SIMULGEL™ NS : | | 0,8% |
| Conservateur : | | 0,2% |

**Exemple 11 : Lait solaire**

| | | |
|---|---|---|
| Composition (EM$_2$) : | | 3,5% |
| LANOL™ 37T : | | 10,0% |
| PARSOL™ MCX : | | 5,0% |
| EUSOLEX™ 4360 : | | 2,0% |
| Eau : | q.s.p. | 100% |
| SEPIPLUS ™ 400 : | | 1,8% |
| Conservateur : | | 0,2% |
| Parfum : | | 0,4% |

**Exemple 16 : Emulsion bronzante sans soleil**

| | | |
|---|---|---|
| LANOL™ 99 : | | 15% |
| Composition (EM$_2$) : | | 5,0% |
| PARSOL™ MCX : | | 3,0% |
| Eau : | q.s.p. | 100% |
| Dihydroxyacétone : | | 5,0% |
| Phosphate monosodique : | | 0,2% |
| SIMULGEL™ NS : | | 2,5% |
| Parfum : | | 0,3% |
| SEPICIDE™ HB : | | 0,8% |
| Hydroxyde de sodium : | q.s. | pH=5. |

**Exemple 17 : Crème de soin**

| | |
|---|---|
| Cyclométhicone : | 10% |
| SIMULGEL™ EG: | 2,8% |
| Composition (EM$_1$) : | 4,5% |

(suite)

| | | |
|---|---|---|
| Conservateur : | | 0,65% |
| Lysine : | | 0,025% |
| EDTA (sel disodique) : | | 0,05% |
| Gomme de xanthane : | | 0,2% |
| Glycérine : | | 3% |
| Eau : | qsp. | 100% |

**Exemple 18 : Crème solaire**

| | | |
|---|---|---|
| SIMULSOL™ 165 : | | 3% |
| Composition (EM$_3$) : | | 2% |
| Benzoate C12-C15 : | | 8% |
| PECOSIL™ PS 100 : | | 2% |
| Diméthicone : | | 2% |
| Cyclométhicone : | | 5% |
| Para-méthoxy cinnamate d'octyle : | | 6% |
| Benzophénone-3 : | | 4% |
| Oxyde de Titane : | | 8% |
| Gomme xanthane : | | 0,2% |
| Butylèneglycol : | | 5% |
| Eau déminéralisée : | qsp | 100% |
| SIMULGEL™ NS : | | 1,5% |
| Conservateur, parfum : | | qs |

**Exemple 19 : Gel solaire et autobronzant**

| | | |
|---|---|---|
| Composition (EM3) : | | 3,0% |
| Triheptanoate de glycéryle : | | 10,0% |
| DEEPALINE™ PVB : | | 1,05% |
| SIMULGEL™ EG : | | 2,2% |
| Eau : | qs | 100% |
| Dihydroxyacétone : | | 5% |
| Parfum : | | 0,1% |
| SEPICIDE™ HB : | | 0,3% |
| SEPICIDE™ CI : | | 0,1% |
| PARSOL™ MCX : | | 4,0% |

Les définitions des produits commerciaux utilisés dans les exemples, sont les suivantes :

Le MICROPEARL™ M 100 est une poudre ultra fine au toucher très doux et à action matifiante commercialisée par la société MATSUMO
Le SEPICIDE™ CI, imidazolidine urée, est un agent conservateur commercialisé par la société SEPPIC.
Le SIMULSOL™ 165 est du stéarate de glycérol auto-émulsionnable commercialisée par la société SEPPIC.
Le SEPICIDE™ HB, qui est un mélange de phénoxyéthanol, de méthylparaben, d'éthylparaben, de propylparaben et de butylparaben, est un agent conservateur commercialisé par la société SEPPIC.
Le PARSOL™ MCX est du para-méthoxy cinnamate d'octyle ; commercialisé par la société GIVAUDAN.
Le LANOL™ 37T est du triheptanoate de glycérol, commercialisé par la société SEPPIC.
Le SOLAGUM™ L est un carraghénane commercialisé par la société SEPPIC.

L'EUSOLEX™ 4360 est un filtre solaire commercialisé par la société MERCK.

La DEEPALINE™ PVB, est un hydrolysât de protéines de blé acylé commercialisé par la société SEPPIC.

Le SIMULGEL™ EG : Latex inverse auto-inversible de copolymère tel que ceux décrits dans la publication internationale WO 99/36445 (Dénomination INCI Sodium acrylate/Sodium acryloyldimethyl taurate copolymer et Isohexadecane et Polysorbate 80) commercialisé par la société SEPPIC ;

SEPIPLUS™ 400 : Latex inverse auto-inversible de copolymères tel que ceux décrits dans la publication internationale WO 2005/040230 (Dénomination INCI : Polyacrylate-13 & Polyisobutene & Polysorbate 20), commercialisé par la société SEPPIC ;

Le SIMULGEL™ NS : Latex inverse auto-inversible de copolymères épaississants (Dénomination INCI : hydroxyéthyl acrylate/Sodium acryloyldimethyl taurate copolymer et Squalane et Polysorbate 60) commercialisé par la société SEPPIC ;

Le SIMULGEL™ INS 100: Latex inverse auto-inversible de copolymères épaississants (Dénomination INCI : hydroxyéthyl acrylate/Sodium acryloyldimethyl taurate copolymer et Isohexadecane et Polysorbate 60) commercialisé par la société SEPPIC ;

Le PRIMOL™ 352 est une huile minérale commercialisée par la société EXXON.

Le PECOSIL™ PS 100 est du Dimethicone PEG-7 commercialisé par la société PHOENIX.

## Revendications

1. Composition (EM) comprenant pour 100% de sa masse :

    **1)** - Une proportion supérieure ou égale à 20% massique et inférieure ou égale à 80% massique d'un agent diluant choisi parmi le 1,3-propanediol, le 1,3-butanediol, le 1,4-butanediol, le 2-méthyl 2,4-pentanediol, le 1,6-hexanediol, le 1,8-octanediol ou un composé de formule (I) :

    $$HO\text{-}[CH_2\text{-}CH(OH)\text{-}CH_2\text{-}O^-]_n H \qquad (I),$$

    dans laquelle n représente un nombre entier supérieur ou égal à 1 et inférieur ou égal à 6, ou d'un mélange desdits agents diluants

    **2)** - Une proportion supérieure ou égale à 20% massique et inférieure ou égale à 80% massique d'une composition (C) comprenant pour 100% de sa masse :

    **a)** - Une proportion supérieure à 0% et inférieure ou égale à 4% massique d'au moins un alcool de formule (II) :

    $$C_m H_{2m+1}\text{-}OH \qquad (II),$$

    dans laquelle m représente un nombre entier pair supérieur ou égal à 8 et inférieur ou égal à 18 ;

    **b)** - Une proportion supérieure ou égale à 96% massique et inférieure à 100% massique d'une composition $(C_1)$ comprenant :

    **$b_1$)** - Une proportion supérieure ou égale à 60% massique et inférieure ou égale 90% massique d'une composition $(C_2)$ comprenant pour 100% de sa masse :

    **$b_{1i}$)** - Une proportion supérieure ou égale à 5% massique et inférieure ou égale à 20% massique d'une composition $(C_{21})$ représentée par la formule (III) :

    $$R_{21}\text{-}O\text{-}(G_{21})_r\text{-}H \qquad (III),$$

    dans laquelle $R_{21}$ représente le radical n-dodécyle, $G_{21}$ représente le reste d'un sucre réducteur et r représente un nombre décimal supérieur ou égal à 1,05 et inférieur ou égal à 5, ladite composition $(C_{21})$ consistant en un mélange des composés de formules $(III_1)$, $(III_2)$, $(III_3)$, $(III_4)$ et $(III_5)$ :

    $$R_{21}\text{-}O\text{-}(G_{21})_1\text{-}H \qquad (III_1),$$

    $$R_{21}\text{-}O\text{-}(G_{21})_2\text{-}H \qquad (III_2),$$

$$R_{21}\text{-}O\text{-}(G_{21})_3\text{-}H \qquad (III_3),$$

$$R_{21}\text{-}O\text{-}(G_{21})_4\text{-}H \qquad (III_4),$$

$$R_{21}\text{-}O\text{-}(G_{21})_5\text{-}H \qquad (III_5),$$

en des proportions molaires en dits composés de formules $(III_1)$, $(III_2)$, $(III_3)$, $(III_4)$ et $(III_5)$ respectivement égales à $a_1$, $a_2$, $a_3$, $a_4$ et $a_5$, telles que la somme $a_1 + a_2 + a_3 + a_4 + a_5$ est égale à 1, et que la somme $a_1 + 2a_2 + 3a_3 + 4a_4 + 5a_5$ est égale à r ;

**b$_{1ii}$)** - Une proportion supérieure ou égale à 10% massique et inférieure ou égale à 20% massique d'une composition $(C_{22})$ représentée par la formule (IV) :

$$R_{22}\text{-}O\text{-}(G_{22})_s\text{-}H \qquad (IV),$$

dans laquelle $R_{22}$ représente le radical n-tétradécyle, $G_{22}$ représente le reste d'un sucre réducteur et s représente un nombre décimal supérieur ou égal à 1,05 et inférieur ou égal à 5, ladite composition $(C_{22})$ consistant en un mélange des composés de formules $(IV_1)$, $(IV_2)$, $(IV_3)$, $(IV_4)$ et $(IV_5)$ :

$$R_{22}\text{-}O\text{-}(G_{22})_1\text{-}H \qquad (IV_1),$$

$$R_{22}\text{-}O\text{-}(G_{22})_2\text{-}H \qquad (IV_2),$$

$$R_{22}\text{-}O\text{-}(G_{22})_3\text{-}H \qquad (IV_3),$$

$$R_{22}\text{-}O\text{-}(G_{22})_4\text{-}H \qquad (IV_4),$$

$$R_{22}\text{-}O\text{-}(G_{22})_5\text{-}H \qquad (IV_5),$$

en des proportions molaires en dits composés de formules $(IV_1)$, $(IV_2)$, $(IV_3)$, $(IV_4)$ et $(IV_5)$ respectivement égales à $b_1$, $b_2$, $b_3$, $b_4$ et $b_5$, telles que la somme: $b_1 + b_2 + b_3 + b_4 + b_5$ est égale à 1, et que la somme $b_1 + 2b_2 + 3b_3 + 4b_4 + 5b_5$ est égale à s ;

**b$_{1iii}$)** - Une proportion supérieure ou égale à 25% massique et inférieure ou égale à 40% massique d'une composition $(C_{23})$ représentée par la formule (V) :

$$R_{23}\text{-}O\text{-}(G_{23})_t\text{-}H \qquad (V),$$

dans laquelle $R_{23}$ représente le radical n-octyle, $G_{23}$ représente le reste d'un sucre réducteur et t représente un nombre décimal supérieur ou égal à 1,05 et inférieur ou égal à 5, ladite composition $(C_{23})$ consistant en un mélange des composés de formules $(V_1)$, $(V_2)$, $(V_3)$, $(V_4)$ et $(V_5)$ :

$$R_{23}\text{-}O\text{-}(G_{23})_1\text{-}H \qquad (V_1),$$

$$R_{23}\text{-}O\text{-}(G_{23})_2\text{-}H \qquad (V_2),$$

$$R_{23}\text{-}O\text{-}(G_{23})_3\text{-}H \qquad (V_3),$$

$$R_{23}\text{-}O\text{-}(G_{23})_4\text{-}H \qquad (V_4),$$

$$R_{23}\text{-}O\text{-}(G_{23})_5\text{-}H \qquad (V_5),$$

en des proportions molaires en dits composés de formules $(V_1)$, $(V_2)$, $(V_3)$, $(V_4)$ et $(V_5)$ respectivement égales à $c_1$, $c_2$, $c_3$, $c_4$ et $c_5$, telles que la somme: $c_1 + c_2 + c_3 + c_4 + c_5$ est égale à 1, et que la somme $c_1 + 2c_2 + 3C_3 + 4c_4 + 5c_5$ est égale à t ;

**b$_{1iv}$)** - Une proportion massique supérieure ou égale à 30% massique et inférieure ou égale à 55% massique d'une composition (C24) représentée par la formule (VI) :

$$R_{24}\text{-}O\text{-}(G_{24})_u\text{-}H \qquad (VI),$$

dans laquelle $R_{24}$ représente le radical n-décyle, $G_{24}$ représente le reste d'un sucre réducteur et u représente un nombre décimal supérieur ou égal à 1,05 et inférieur ou égal à 5, ladite composition $(C_{24})$ consistant en un mélange des composés de formules $(VI_1)$, $(VI_2)$, $(VI_3)$, $(VI_4)$ et $(VI_5)$ :

$$R_{24}\text{-O-}(G_{24})_1\text{-H} \qquad (VI_1),$$

$$R_{24}\text{-O-}(G_{24})_2\text{-H} \qquad (VI_2),$$

$$R_{24}\text{-O-}(G_{24})_3\text{-H} \qquad (VI_3),$$

$$R_{24}\text{-O-}(G_{24})_4\text{-H} \qquad (VI_4),$$

$$R_{24}\text{-O-}(G_{24})_5\text{-H} \qquad (VI_5),$$

en dans les proportions molaires en dits composés de formules $(VI_1)$, $(VI_2)$, $(VI_3)$, $(VI_4)$ et $(VI_5)$ respectivement égales à $d_1$, $d_2$, $d_3$, $d_4$ et $d_5$, telles que la somme: $d_1 + d_2 + d_3 + d_4 + d_5$ est égale à 1, et que la somme $d_1 + 2d_2 + 3d_3 + 4d_4 + 5d_5$ est égale à u ; étant entendu que la somme des proportions massiques des compositions $(C_{21})$, $(C_{22})$, $(C_{23})$, et $(C_{24})$, est égale à 100% ;

$b_2)$ - D'une proportion supérieure ou égale à 10% massique et inférieure ou égale à 40% massique d'une composition $(C_3)$ comprenant pour 100% de sa masse :

$b_{2i})$ - Une proportion supérieure ou égale à 30% massique et inférieure ou égale à 50% massique d'une composition $(C_{31})$ représentée par la formule (VII) :

$$R_{31}\text{-O-}(G_{31})_x\text{-H} \qquad (VII)$$

dans laquelle $R_{31}$ représente le radical n-hexadécyle, $G_{31}$ représente le reste d'un sucre réducteur et x représente un nombre décimal supérieur ou égal à 1,05 et inférieur ou égal à 5, ladite composition $(C_{31})$ consistant en un mélange des composés de formules $(VII_1)$, $(VII_2)$, $(VII_3)$, $(VII_4)$ et $(VII_5)$ :

$$R_{31}\text{-O-}(G_{31})_1\text{-H} \qquad (VII_1),$$

$$R_{31}\text{-O-}(G_{31})_2\text{-H} \qquad (VII_2),$$

$$R_{31}\text{-O-}(G_{31})_3\text{-H} \qquad (VII_3),$$

$$R_{31}\text{-O-}(G_{31})_4\text{-H} \qquad (VII_4),$$

$$R_{31}\text{-O-}(G_{31})_5\text{-H} \qquad (VII_5),$$

en des proportions molaires en dits composés de formules $(VII_1)$, $(VII_2)$, $(VII_3)$, $(VII_4)$ et $(VII_5)$ respectivement égales $a'_1$, $a'_2$, $a'_3$, $a'_4$ et $a'_5$, telles que la somme $a'_1 + a'_2 + a'_3 + a'_4 + a'_5$ est égale à 1, et que la somme $a'_1 + 2a'_2 + 3a'_3 + 4a'_4 + 5a'_5$ est égale à x ;

$b_{2ii})$ - Une proportion supérieure ou égale à 50% massique et inférieure ou égale à 70% massique d'une composition $(C_{32})$ représentée par la formule (VIII) :

$$R_{32}\text{-O-}(G_{32})_y\text{-H} \qquad (VIII),$$

dans laquelle $R_{32}$ représente le radical n-octadécyle, $G_{32}$ représente le reste d'un sucre réducteur et y représente un nombre décimal supérieur ou égal à 1,05 et inférieur ou égal à 5, ladite composition $(C_{32})$ consistant en un mélange des composés de formules $(VIII_1)$, $(VIII_2)$, $(VIII_3)$, $(VIII_4)$ et $(VIII_5)$ :

$$R_{32}\text{-O-}(G_{32})_1\text{-H} \qquad (VIII_1),$$

$$R_{32}\text{-O-}(G_{32})_2\text{-H} \qquad (VIII_2),$$

$$R_{32}\text{-O-}(G_{32})_3\text{-H} \qquad (VIII_3),$$

$$R_{32}\text{-O-}(G_{32})_4\text{-H} \qquad (VIII_4),$$

$$R_{32}\text{-O-}(G_{32})_5\text{-H} \qquad (VIII_5),$$

En des proportions molaires en dits composés de formules $(VIII_1)$, $(VIII_2)$, $(VIII_3)$, $(VIII_4)$ et $(VIII_5)$ respectivement égales à $b'_1$, $b'_2$, $b'_3$, $b'_4$ et $b'_5$, telles que la somme $b'_1 + b'_2 + b'_3 + b'_4 + b'_5$ est égale à 1, et que la somme $b'_1 + 2b'_2 + 3b'_3 + 4b'_4 + 5b'_5$ est égale à y, étant entendu que la somme des proportions massiques des compositions $(C_{31})$ et $(C_{32})$ est égale à 100%.

2. Composition (EM) telle que définie à la revendication 1, **caractérisée en ce que** dans les formules (III), (IV), (V) et (VI), lesdits restes d'un sucre réducteur $G_{21}$, $G_{22}$, $G_{23}$, $G_{24}$, représentent un même reste de sucre réducteur choisi parmi le reste du glucose, le reste du xylose et le reste de l'arabinose.

3. Composition (EM) telle que définie à la revendication 2, **caractérisée en ce que** dans les formules (III), (IV), (V) et (VI), lesdits restes d'un sucre réducteur $G_{21}$, $G_{22}$, $G_{23}$, G24, représentent le reste du glucose.

4. Composition (EM) telle que définie à la revendication 2, **caractérisée en ce que** dans les formules (III), (IV), (V) et (VI), lesdits restes d'un sucre réducteur $G_{21}$, $G_{22}$, $G_{23}$, $G_{24}$, représentent le reste du xylose.

5. Composition (EM) telle que définie à l'une ou quelconque des revendications 1 à 4, **caractérisée en ce que** dans les formules (III), (IV), (V) et (VI), r, s, t et u respectivement, représentent indépendamment l'un de l'autre un nombre décimal supérieur ou égal à 1,05 et inférieur ou égal à 2,5.

6. Composition (EM) telle que définie à l'une ou quelconque des revendications 1 à 5, **caractérisée en ce que** dans les formules (VII) et (VIII), lesdits restes d'un sucre réducteur $G_{31}$ et $G_{32}$ représentent un même reste de sucre réducteur choisi parmi le reste du glucose, le reste du xylose et le reste de l'arabinose.

7. Composition (EM) telle que définie à la revendication 6, **caractérisée en ce que** dans les formules (VII) et (VIII), lesdits restes d'un sucre réducteur $G_{31}$ et $G_{32}$ représente le reste du glucose.

8. Composition (EM) telle que définie à la revendication 6, **caractérisée en ce que** dans les formules (VII) et (VIII), lesdits restes d'un sucre réducteur $G_{31}$ et $G_{32}$ représente le reste du xylose.

9. Composition (EM) telle que définie à l'une ou quelconque des revendications 1 à 8, **caractérisée en ce que** dans les formules (VII) et (VIII), x et y respectivement, représentent un nombre décimal supérieur ou égal à 1,05 et inférieur ou égal à 2,5.

10. Composition (EM) telle que définie à l'une ou quelconque des revendications 1 à 9, **caractérisée en ce que** le ratio massique :

$$\Delta = \text{Masse de la composition } (C_2)/\text{Masse de la composition } (C_3),$$

est supérieur ou égal à 1 et inférieur ou égal à 10.

11. Composition (EM) telle que définie à l'une ou quelconque des revendications 1 à 10, **caractérisée en ce que** l'agent diluant est le 1,3-propanediol ou le glycérol.

12. Composition (EM) telle que définie à l'une ou quelconque des revendications 1 à 11, **caractérisée en ce qu'**elle comprend en outre
3) -Jusqu'à 7% massique d'eau,
étant entendu que la somme des proportions massiques dudit agent diluant, de ladite composition (C) et de l'eau est égale à 100%.

**13.** Procédé de préparation de la composition (EM) telle que définie à l'une ou quelconque des revendications 1 à 12, comprenant les étapes successives suivantes :

- Une <u>étape A)</u> de réaction, dans les proportions souhaitées, d'un sucre réducteur de formule (IX) ou un mélange de sucres réducteurs de formule (IX) :

$$HO\text{-}(G)\text{-}H \qquad (IX)$$

dans laquelle G représente le reste d'un sucre réducteur, avec un excès molaire d'un mélange d'alcools de formule (II) :

$$C_mH_{2m+1}\text{-}OH \qquad (II),$$

telle que définie précédemment, pour former un mélange de composés de formule (III), (IV), (V), (VI), (VII) et (VIII) et d'un excès dudit mélange d'alcools de formule (II),
- Une <u>étape B)</u> d'élimination partielle de l'excès dudit mélange d'alcools de formule (II) pour former ladite composition (C) telle que définie précédemment ;
- une <u>étape C)</u> de mélange de ladite composition (C), avec au moins un agent diluant tel que défini précédemment, et si nécessaire ou si désiré,
- une <u>étape D</u> d'addition d'eau.

**14.** Utilisation d'une composition (EM), telle que définie à l'une ou quelconque des revendications 1 à 12, comme agent émulsionnant pour la préparation d'une émulsion fluide de type huile-dans-eau à usage topique.

**15.** Emulsion de type huile-dans-eau (E), comprenant :

- de 50% à 80% massique d'au moins une phase aqueuse ($P_1$) cosmétiquement acceptable,
- de 0,5% à 15% massique d'au moins une composition (EM) telle que définie à l'une quelconque des revendications 1 à 12, et
- de 5% à 49,5% massique d'au moins une phase grasse ($P_2$),

étant entendu que la somme des proportions massiques de ladite phase aqueuse ($P_1$), de ladite composition (EM) et de ladite phase grasse ($P_2$) est égale à 100%.

**Patentansprüche**

**1.** Zusammensetzung (EM), umfassend pro 100 % ihrer Masse:

1) - einen Anteil größer oder gleich 20 Massen-% und kleiner oder gleich 80 Massen-% eines Verdünnungsmittels, das aus 1,3-Propandiol, 1,3-Butandiol, 1,4-Butandiol, 2-Methyl-2,4-pentandiol, 1,6-Hexandiol, 1,8-Octandiol oder einer Verbindung der Formel (I):

$$HO\text{-}[CH_2\text{-}CH(OH)\text{-}CH_2\text{-}O\text{-}]_nH \qquad (I)$$

ausgewählt ist, wobei n für eine ganze Zahl größer oder gleich 1 und kleiner oder gleich 6 steht, oder einer Mischung der Verdünnungsmittel,
2) - einen Anteil größer oder gleich 20 Massen-% und kleiner oder gleich 80 Massen-% einer Zusammensetzung (C), umfassend pro 100 % ihrer Masse:

a) - einen Anteil größer 0 Massen-% und kleiner oder gleich 4 Massen-% mindestens eines Alkohols der Formel (II):

$$C_mH_{2m+1}\text{-}OH \qquad (II),$$

wobei m für eine gerade ganze Zahl größer oder gleich 8 und kleiner oder gleich 18 steht;
b) - einen Anteil größer oder gleich 96 Massen-% und kleiner oder gleich 100 Massen-% einer Zusammensetzung ($C_1$), umfassend:

27

$b_1$) - einen Anteil größer oder gleich 60 Massen-% und kleiner oder gleich 90 Massen-% einer Zusammensetzung ($C_2$), umfassend pro 100 % ihrer Masse:

$b_{1i}$) - einen Anteil größer oder gleich 5 Massen-% und kleiner oder gleich 20 Massen-% einer Zusammensetzung ($C_{21}$), die durch Formel (III) wiedergegeben wird:

$$R_{21}\text{-O-}(G_{21})_r\text{-H} \qquad \text{(III)},$$

wobei $R_{21}$ für den n-Dodecylrest steht, $G_{21}$ für den Rest eines reduzierenden Zuckers steht und r für eine Dezimalzahl größer oder gleich 1,05 und kleiner oder gleich 5 steht, wobei die Zusammensetzung ($C_{21}$) aus einem Gemisch von Verbindungen der Formeln (III$_1$), (III$_2$), (III$_3$), (III$_4$) und (III$_5$) :

$$R_{21}\text{-O-}(G_{21})_1\text{-H} \qquad \text{(III}_1\text{)},$$

$$R_{21}\text{-O-}(G_{21})_2\text{-H} \qquad \text{(III}_2\text{)},$$

$$R_{21}\text{-O-}(G_{21})_3\text{-H} \qquad \text{(III}_3\text{)},$$

$$R_{21}\text{-O-}(G_{21})_4\text{-H} \qquad \text{(III}_4\text{)},$$

$$R_{21}\text{-O-}(G_{21})_5\text{-H} \qquad \text{(III}_5\text{)},$$

in solchen molaren Anteilen der Verbindungen der Formeln (III$_1$), (III$_2$), (III$_3$), (III$_4$) und (III$_5$), die gleich $a_1$, $a_2$, $a_3$, $a_4$ bzw. $a_5$ sind, besteht, dass die Summe $a_1 + a_2 + a_3 + a_4 + a_5$ gleich 1 ist und dass die Summe $a_1 + 2a_2 + 3a_3 + 4a_4 + 5a_5$ gleich r ist;

$b_{1ii}$) - einen Anteil größer oder gleich 10 Massen-% und kleiner oder gleich 20 Massen-% einer Zusammensetzung ($C_{22}$), die durch Formel (IV) wiedergegeben wird:

$$R_{22}\text{-O-}(G_{22})_s\text{-H} \qquad \text{(IV)},$$

wobei $R_{22}$ für den n-Tetradecylrest steht, $G_{22}$ für den Rest eines reduzierenden Zuckers steht und s für eine Dezimalzahl größer oder gleich 1,05 und kleiner oder gleich 5 steht, wobei die Zusammensetzung ($C_{22}$)aus einem Gemisch von Verbindungen der Formeln (IV$_1$), (IV$_2$), (IV$_3$), (IV$_4$) und (IV$_5$) :

$$R_{22}\text{-O-}(G_{22})_1\text{-H} \qquad \text{(IV}_1\text{)},$$

$$R_{22}\text{-O-}(G_{22})_2\text{-H} \qquad \text{(IV}_2\text{)},$$

$$R_{22}\text{-O-}(G_{22})_3\text{-H} \qquad \text{(IV}_3\text{)},$$

$$R_{22}\text{-O-}(G_{22})_4\text{-H} \qquad \text{(IV}_4\text{)},$$

$$R_{22}\text{-O-}(G_{22})_5\text{-H} \qquad \text{(IV}_5\text{)},$$

in solchen molaren Anteilen der Verbindungen der Formeln (IV$_1$), (IV$_2$), (IV$_3$), (IV$_4$) und (IV$_5$), die gleich $b_1$, $b_2$, $b_3$, $b_4$ bzw. $b_5$ sind, besteht, dass die Summe $b_1 + b_2 + b_3 + b_4 + b_5$ gleich 1 ist und dass die Summe $b_1 + 2b_2 + 3b_3 + 4b_4 + 5b_5$ gleich s ist;

$b_{1iii}$) - einen Anteil größer oder gleich 25 Massen-% und kleiner oder gleich 40 Massen-% einer Zusammensetzung ($C_{23}$), die durch Formel (V) wiedergegeben wird:

$$R_{23}\text{-O-}(G_{23})_t\text{-H} \qquad \text{(V)},$$

wobei $R_{23}$ für den n-Octylrest steht, $G_{23}$ für den Rest eines reduzierenden Zuckers steht und t für eine Dezimalzahl größer oder gleich 1,05 und kleiner oder gleich 5 steht, wobei die Zusammensetzung ($C_{23}$) aus einem Gemisch von Verbindungen der Formeln (V$_1$), (V$_2$), (V$_3$), (V$_4$) und (V$_5$) :

$$R_{23}\text{-O-}(G_{23})_1\text{-H} \qquad (V_1),$$

$$R_{23}\text{-O-}(G_{23})_2\text{-H} \qquad (V_2),$$

$$R_{23}\text{-O-}(G_{23})_3\text{-H} \qquad (V_3),$$

$$R_{23}\text{-O-}(G_{23})_4\text{-H} \qquad (V_4),$$

$$R_{23}\text{-O-}(G_{23})_5\text{-H} \qquad (V_5),$$

in solchen molaren Anteilen der Verbindungen der Formeln $(V_1)$, $(V_2)$, $(V_3)$, $(V_4)$ und $(V_5)$, die gleich $c_1$, $c_2$, $c_3$, $c_4$ bzw. $c_5$ sind, besteht, dass die Summe $c_1 + c_2 + c_3 + c_4 + c_5$ gleich 1 ist und dass die Summe $c_1 + 2c_2 + 3c_3 + 4c_4 + 5c_5$ gleich t ist;

$b_{1iv}$) - einen Massenanteil größer oder gleich 30 Massen-% und kleiner oder gleich 55 Massen-% einer Zusammensetzung $(C_{24})$, die durch Formel (VI) wiedergegeben wird:

$$R_{24}\text{-O-}(G_{24})_u\text{-H} \qquad (VI),$$

wobei $R_{24}$ für den n-Decylrest steht, $G_{24}$ für den Rest eines reduzierenden Zuckers steht und u für eine Dezimalzahl größer oder gleich 1,05 und kleiner oder gleich 5 steht, wobei die Zusammensetzung $(C_{24})$ aus einem Gemisch von Verbindungen der Formeln $(VI_1)$, $(VI_2)$, $(VI_3)$, $(VI_4)$ und $(VI_5)$ :

$$R_{24}\text{-O-}(G_{24})_1\text{-H} \qquad (VI_1),$$

$$R_{24}\text{-O-}(G_{24})_2\text{-H} \qquad (VI_2),$$

$$R_{24}\text{-O-}(G_{24})_3\text{-H} \qquad (VI_3),$$

$$R_{24}\text{-O-}(G_{24})_4\text{-H} \qquad (VI_4),$$

$$R_{24}\text{-O-}(G_{24})_5\text{-H} \qquad (VI_5),$$

in solchen molaren Anteilen der Verbindungen der Formeln $(VI_1)$, $(VI_2)$, $(VI_3)$, $(VI_4)$ und $(VI_5)$, die gleich $d_1$, $d_2$, $d_3$, $d_4$ bzw. $d_5$ sind, besteht, dass die Summe $d_1 + d_2 + d_3 + d_4 + d_5$ gleich 1 ist und dass die Summe $d_1 + 2d_2 + 3d_3 + 4d_4 + 5d_5$ gleich u ist;

mit der Maßgabe, dass die Summe der Massenanteile der Zusammensetzungen $(C_{21})$, $(C_{22})$, $(C_{23})$ und $(C_{24})$ gleich 100 % ist;

b2) - einen Anteil größer oder gleich 10 Massen-% und kleiner oder gleich 40 Massen-% einer Zusammensetzung $(C_3)$, umfassend pro 100 % ihrer Masse:

$b_{2i}$) - einen Anteil größer oder gleich 30 Massen-% und kleiner oder gleich 50 Massen-% einer Zusammensetzung $(C_{31})$, die durch Formel (VII) wiedergegeben wird:

$$R_{31}\text{-O-}(G_{31})_x\text{-H} \qquad (VII),$$

wobei $R_{31}$ für den n-Hexadecylrest steht, $G_{31}$ für den Rest eines reduzierenden Zuckers steht und x für eine Dezimalzahl größer oder gleich 1,05 und kleiner oder gleich 5 steht, wobei die Zusammensetzung $(C_{31})$ aus einem Gemisch von Verbindungen der Formeln $(VII_1)$, $(VII_2)$, $(VII_3)$, $(VII_4)$ und $(VII_5)$ :

$$R_{31}\text{-O-}(G_{31})_1\text{-H} \qquad (VII_1),$$

$$R_{31}\text{-O-}(G_{31})_2\text{-H} \qquad (VII_2),$$

$$R_{31}\text{-O-}(G_{31})_3\text{-H} \qquad (VII_3),$$

$$R_{31}\text{-}O\text{-}(G_{31})_4\text{-}H \qquad (VII_4),$$

$$R_{31}\text{-}O\text{-}(G_{31})_5\text{-}H \qquad (VII_5),$$

in solchen molaren Anteilen der Verbindungen der Formeln $(VII_1)$, $(VII_2)$, $(VII_3)$, $(VII_4)$ und $(VII_5)$, die gleich $a'_1$, $a'_2$, $a'_3$, $a'_4$ bzw. $a'_5$ sind, besteht, dass die Summe $a'_1 + a'_2 + a'_3 + a'_4 + a'_5$ gleich 1 ist und dass die Summe $a'_1 + 2a'_2 + 3a'_3 + 4a'_4 + 5a'_5$ gleich x ist;

$b_{2ii}$) - einen Anteil größer oder gleich 50 Massen-% und kleiner oder gleich 70 Massen-% einer Zusammensetzung $(C_{32})$, die durch Formel (VIII) wiedergegeben wird:

$$R_{32}\text{-}O\text{-}(G_{32})_y\text{-}H \qquad (VIII),$$

wobei $R_{32}$ für den n-Octadecylrest steht, $G_{32}$ für den Rest eines reduzierenden Zuckers steht und y für eine Dezimalzahl größer oder gleich 1,05 und kleiner oder gleich 5 steht, wobei die Zusammensetzung $(C_{32})$ aus einem Gemisch von Verbindungen der Formeln $(VIII_1)$, $(VIII_2)$, $(VIII_3)$, $(VIII_4)$ und $(VIII_5)$ :

$$R_{32}\text{-}O\text{-}(G_{32})_1\text{-}H \qquad (VIII_1),$$

$$R_{32}\text{-}O\text{-}(G_{32})_2\text{-}H \qquad (VIII_2),$$

$$R_{32}\text{-}O\text{-}(G_{32})_3\text{-}H \qquad (VIII_3),$$

$$R_{32}\text{-}O\text{-}(G_{32})_4\text{-}H \qquad (VIII_4),$$

$$R_{32}\text{-}O\text{-}(G_{32})_5\text{-}H \qquad (VIII_5),$$

in solchen molaren Anteilen der Verbindungen der Formeln $(VIII_1)$, $(VIII_2)$, $(VIII_3)$, $(VIII_4)$ und $(VIII_5)$, die gleich $b'_1$, $b'_2$, $b'_3$, $b'_4$ bzw. $b'_5$ sind, besteht, dass die Summe $b'_1 + b'_2 + b'_3 + b'_4 + b'_5$ gleich 1 ist und dass die Summe $b'_1 + 2b'_2 + 3b'_3 + 4b'_4 + 5b'_5$ gleich y ist, mit der Maßgabe, dass die Summe der Massenanteile der Zusammensetzungen $(C_{31})$ und $(C_{32})$ gleich 100 % ist.

2. Zusammensetzung (EM) gemäß Anspruch 1, **dadurch gekennzeichnet, dass** in den Formeln (III), (IV), (V) und (VI) die Reste eines reduzierenden Zuckers $G_{21}$, $G_{22}$, $G_{23}$ und $G_{24}$ für den gleichen Rest eines reduzierenden Zuckers, der aus dem Glucoserest, dem Xyloserest und dem Arabinoserest ausgewählt ist, stehen.

3. Zusammensetzung (EM) gemäß Anspruch 2, **dadurch gekennzeichnet, dass** in den Formeln (III), (IV), (V) und (VI) die Reste eines reduzierenden Zuckers $G_{21}$, $G_{22}$, $G_{23}$ und $G_{24}$ für den Glucoserest stehen.

4. Zusammensetzung (EM) gemäß Anspruch 2, **dadurch gekennzeichnet, dass** in den Formeln (III), (IV), (V) und (VI) die Reste eines reduzierenden Zuckers $G_{21}$, $G_{22}$, $G_{23}$ und $G_{24}$ für den Xyloserest stehen.

5. Zusammensetzung (EM) gemäß einem der Ansprüche 1 bis 4, **dadurch gekennzeichnet, dass** in den Formeln (III), (IV), (V) und (VI) r, s, t und u jeweils unabhängig voneinander für eine Dezimalzahl größer oder gleich 1,05 und kleiner oder gleich 2,5 stehen.

6. Zusammensetzung (EM) gemäß einem der Ansprüche 1 bis 5, **dadurch gekennzeichnet, dass** in den Formeln (VII) und (VIII) die Reste eines reduzierenden Zuckers $G_{31}$ und $G_{32}$ für den gleichen Rest eines reduzierenden Zuckers, der aus dem Glucoserest, dem Xyloserest und dem Arabinoserest ausgewählt ist, stehen.

7. Zusammensetzung (EM) gemäß Anspruch 6, **dadurch gekennzeichnet, dass** in den Formeln (VII) und (VIII) die Reste eines reduzierenden Zuckers $G_{31}$ und $G_{32}$ für den Glucoserest stehen.

8. Zusammensetzung (EM) gemäß Anspruch 6, **dadurch gekennzeichnet, dass** in den Formeln (VII) und (VIII) die Reste eines reduzierenden Zuckers $G_{31}$ und $G_{32}$ für den Xyloserest stehen.

9. Zusammensetzung (EM) gemäß einem der Ansprüche 1 bis 8, **dadurch gekennzeichnet, dass** in den Formeln (VII) und (VIII) x und y jeweils für eine Dezimalzahl größer oder gleich 1,05 und kleiner oder gleich 2,5 stehen.

10. Zusammensetzung (EM) gemäß einem der Ansprüche 1 bis 9, **dadurch gekennzeichnet, dass** das Massenverhältnis:

$$\Delta = \text{Masse von Zusammensetzung } (C_2)/\text{Masse von Zusammensetzung } (C_3)$$

größer oder gleich 1 und kleiner oder gleich 10 ist.

11. Zusammensetzung (EM) gemäß einem der Ansprüche 1 bis 10, **dadurch gekennzeichnet, dass** es sich bei dem Verdünnungsmittel um 1,3-Propandiol oder Glycerin handelt.

12. Zusammensetzung (EM) gemäß einem der Ansprüche 1 bis 11, **dadurch gekennzeichnet, dass** sie außerdem 3) - bis zu 7 Massen-% Wasser umfasst, wobei sich versteht, dass die Summe der Massenanteile des Verdünnungsmittels, der Zusammensetzung (C) und des Wassers gleich 100 % ist.

13. Verfahren zur Herstellung der Zusammensetzung (EM) gemäß einem der Ansprüche 1 bis 12, das die folgenden aufeinanderfolgenden Schritte umfasst:

- einen Schritt A) der Umsetzung eines reduzierenden Zuckers der Formel (IX) oder eines Gemisches von reduzierenden Zuckers der Formel Formel (IX):

$$\text{HO-(G)-H} \qquad (IX),$$

wobei G für den Rest eines reduzierenden Zuckers steht, mit einem molaren Überschuss eines Gemischs von Alkoholen der Formel (II):

$$C_mH_{2m+1}\text{-OH} \qquad (II)$$

gemäß obiger Definition in den gewünschten Anteilen zur Bildung eines Gemischs von Verbindungen der Formel (III), (IV), (V), (VI), (VII) und (VIII) und eines Überschusses des Gemischs von Alkoholen der Formel (II),
- einen Schritt B) der teilweisen Entfernung des Überschusses des Gemischs von Alkoholen der Formel (II) zur Bildung der Zusammensetzung (C) gemäß obiger Definition;
- einen Schritt C) des Mischens der Zusammensetzung (C) mit mindestens einem Verdünnungsmittel gemäß obiger Definition und, falls notwendig oder falls gewünscht,
- einen Schritt D der Zugabe von Wasser.

14. Verwendung einer Zusammensetzung (EM) gemäß einem der Ansprüche 1 bis 12 als Emulgator für die Herstellung einer Fluidemulsion vom Öl-in-Wasser-Typ zur topischen Verwendung.

15. Emulsion vom Öl-in-Wasser-Typ (E), umfassend:

- 50 bis 80 Massen-% mindestens einer kosmetisch unbedenklichen wässrigen Phase ($P_1$),
- 0,5 bis 15 Massen-% mindestens einer Zusammensetzung (EM) gemäß einem der Ansprüche 1 bis 12 und
- 5 bis 49,5 Massen-% mindestens einer Fettphase ($P_2$),

wobei es sich versteht, dass die Summe der Massenanteile der wässrigen Phase ($P_1$), der Zusammensetzung (EM) und der Fettphase ($P_2$) gleich 100 % ist.

**Claims**

1. Composition (EM) comprising, per 100% of its mass:

   **1)** - A proportion of greater than or equal to 20% by mass and less than or equal to 80% by mass of a diluent chosen from 1,3-propanediol, 1,3-butanediol, 1,4-butanediol, 2-methyl-2,4-pentanediol, 1,6-hexanediol and 1,8-octanediol or a compound of formula (I):

$$HO-[CH_2-CH(OH)-CH_2-O-]_nH \qquad (I),$$

in which n represents an integer of greater than or equal to 1 and less than or equal to 6, or a mixture of said diluents;

**2)** - A proportion of greater than or equal to 20% by mass and less than or equal to 80% by mass of a composition (C) comprising, per 100% of its mass:

**a)** - A proportion of greater than 0% and less than or equal to 4% by mass of at least one alcohol of formula (II):

$$C_mH_{2m+1}-OH \qquad (II),$$

in which m represents an even integer of greater than or equal to 8 and less than or equal to 18;

**b)** - A proportion of greater than or equal to 96% by mass and less than 100% by mass of a composition $(C_1)$ comprising:

**$b_1$)** - A proportion of greater than or equal to 60% by mass and less than or equal to 90% by mass of a composition $(C_2)$ comprising, per 100% of its mass:

**$b_{1i}$)** - A proportion of greater than or equal to 5% by mass and less than or equal to 20% by mass of a composition $(C_{21})$ represented by formula (III) :

$$R_{21}-O-(G_{21})_r-H \qquad (III),$$

in which $R_{21}$ represents an n-dodecyl radical, $G_{21}$ represents a reducing sugar residue and r represents a decimal number greater than or equal to 1.05 and less than or equal to 5, said composition $(C_{21})$ consisting of a mixture of compounds of formulae $(III_1)$, $(III_2)$, $(III_3)$, $(III_4)$ and $(III_5)$ :

$$R_{21}-O-(G_{21})_1-H \qquad (III_1),$$

$$R_{21}-O-(G_{21})_2-H \qquad (III_2),$$

$$R_{21}-O-(G_{21})_3-H \qquad (III_3),$$

$$R_{21}-O-(G_{21})_4-H \qquad (III_4),$$

$$R_{21}-O-(G_{21})_5-H \qquad (III_5),$$

in molar proportions of said compounds of formulae $(III_1)$, $(III_2)$, $(III_3)$, $(III_4)$ and (IIIs) respectively equal to $a_1$, $a_2$, $a_3$, $a_4$ and $a_5$, such that the sum $a_1 + a_2 + a_3 + a_4 + as$ is equal to 1, and such that the sum $a_1 + 2a_2 + 3a_3 + 4a_4 + 5as$ is equal to r;

**$b_{1ii}$)** - A proportion of greater than or equal to 10% by mass and less than or equal to 20% by mass of a composition $(C_{22})$ represented by formula (IV):

$$R_{22}-O-(G_{22})_s-H \qquad (IV),$$

in which $R_{22}$ represents an n-tetradecyl radical, $G_{22}$ represents a reducing sugar residue and s represents a decimal number greater than or equal to 1.05 and less than or equal to 5, said composition $(C_{22})$ consisting of a mixture of compounds of formulae $(IV_1)$, $(IV_2)$, $(IV_3)$, $(IV_4)$ and $(IV_5)$ :

$$R_{22}-O-(G_{22})_1-H \qquad (IV_1),$$

$$R_{22}-O-(G_{22})_2-H \qquad (IV_2),$$

$$R_{22}-O-(G_{22})_3-H \qquad (IV_3),$$

$$R_{22}\text{-O-}(G_{22})_4\text{-H} \qquad (IV_4),$$

$$R_{22}\text{-O-}(G_{22})s\text{-H} \qquad (IV_5),$$

in molar proportions of said compounds of formulae $(IV_1)$, $(IV_2)$, $(IV_3)$, $(IV_4)$ and $(IV_5)$ respectively equal to $b_1$, $b_2$, $b_3$, $b_4$ and bs, such that the sum $b_1 + b_2 + b_3 + b_4 + b_5$ is equal to 1, and such that the sum $b_1 + 2b_2 + 3b_3 + 4b_4 + 5b_5$ is equal to s;

**$b_{1iii}$)** - A proportion of greater than or equal to 25% by mass and less than or equal to 40% by mass of a composition $(C_{23})$ represented by formula (V):

$$R_{23}\text{-O-}(G_{23})_t\text{-H} \qquad (V),$$

in which $R_{23}$ represents an n-octyl radical, $G_{23}$ represents a reducing sugar residue and t represents a decimal number greater than or equal to 1.05 and less than or equal to 5, said composition $(C_{23})$ consisting of a mixture of the compounds of formulae $(V_1)$, $(V_2)$, $(V_3)$, $(V_4)$ and $(V_5)$ :

$$R_{23}\text{-O-}(G_{23})_1\text{-H} \qquad (V_1),$$

$$R_{23}\text{-O-}(G_{23})_2\text{-H} \qquad (V_2),$$

$$R_{23}\text{-O-}(G_{23})_3\text{-H} \qquad (V_3),$$

$$R_{23}\text{-O-}(G_{23})_4\text{-H} \qquad (V_4),$$

$$R_{23}\text{-O-}(G_{23})_5\text{-H} \qquad (V_5),$$

in molar proportions of said compounds of formulae $(V_1)$, $(V_2)$, $(V_3)$, $(V_4)$ and $(V_5)$ respectively equal to $c_1$, $c_2$, $c_3$, $c_4$ and $c_5$, such that the sum $c_1 + c_2 + c_3 + c_4 + c_5$ is equal to 1, and such that the sum $c_1 + 2c_2 + 3c_3 + 4c_4 + 5c_5$ is equal to t;

**$b_{1iv}$)** - A mass proportion of greater than or equal to 30% by mass and less than or equal to 55% by mass of a composition $(C_{24})$ represented by formula (VI):

$$R_{24}\text{-O-}(G_{24})_u\text{-H} \qquad (VI),$$

in which $R_{24}$ represents an n-decyl radical, $G_{24}$ represents a reducing sugar residue and u represents a decimal number greater than or equal to 1.05 and less than or equal to 5, said composition $(C_{24})$ consisting of a mixture of the compounds of formulae $(VI_1)$, $(VI_2)$, $(VI_3)$, $(VI_4)$ and $(VI_5)$ :

$$R_{24}\text{-O-}(G_{24})_1\text{-H} \qquad (VI_1),$$

$$R_{24}\text{-O-}(G_{24})_2\text{-H} \qquad (VI_2),$$

$$R_{24}\text{-O-}(G_{24})_3\text{-H} \qquad (VI_3),$$

$$R_{24}\text{-O-}(G_{24})_4\text{-H} \qquad (VI_4),$$

$$R_{24}\text{-O-}(G_{24})_5\text{-H} \qquad (VI_5),$$

in molar proportions of said compounds of formulae $(VI_1)$ , $(VI_2)$, $(VI_3)$, $(VI_4)$ and $(VI_5)$ respectively equal to $d_1$, $d_2$, $d_3$, $d_4$ and $d_5$, such that the sum $d_1 + d_2 + d_3 + d_4 + d_5$ is equal to 1, and such that the sum $d_1 + 2d_2 + 3d_3 + 4d_4 + 5ds$ is equal to u;

it being understood that the sum of the mass proportions of compositions $(C_{21})$, $(C_{22})$, $(C_{23})$ and $(C_{24})$ is equal to 100%;

**$b_2$)** - A proportion of greater than or equal to 10% by mass and less than or equal to 40% by mass of a composition $(C_3)$ comprising, per 100% of its mass:

**$b_{2i}$)** - A proportion of greater than or equal to 30% by mass and less than or equal to 50% by mass of

a composition ($C_{31}$) represented by formula (VII):

$$R_{31}-O-(G_{31})_x-H \qquad (VII)$$

in which $R_{31}$ represents an n-hexadecyl radical, $G_{31}$ represents a reducing sugar residue and x represents a decimal number greater than or equal to 1.05 and less than or equal to 5, said composition ($C_{31}$) consisting of a mixture of compounds of formulae ($VII_1$), ($VII_2$), ($VII_3$), ($VII_4$) and ($VII_5$):

$$R_{31}-O-(G_{31})_1-H \qquad (VII_1),$$

$$R_{31}-O-(G_{31})_2-H \qquad (VII_2),$$

$$R_{31}-O-(G_{31})_3-H \qquad (VII_3),$$

$$R_{31}-O-(G_{31})_4-H \qquad (VII_4),$$

$$R_{31}-O-(G_{31})_5-H \qquad (VII_5),$$

in molar proportions of said compounds of formulae ($VII_1$), ($VII_2$), ($VII_3$), ($VII_4$) and ($VII_5$) respectively equal to $a'_1$, $a'_2$, $a'_3$, $a'_4$ and $a'_5$, such that the sum $a'_1 + a'_2 + a'_3 + a'_4 + a'_5$ is equal to 1, and such that the sum $a'_1 + 2a'_2 + 3a'_3 + 4a'_4 + 5a'_5$ is equal to x;

**$b_{2ii}$)** - A proportion of greater than or equal to 50% by mass and less than or equal to 70% by mass of a composition ($C_{32}$) represented by formula (VIII):

$$R_{32}-O-(G_{32})_y-H \qquad (VIII) ,$$

in which $R_{32}$ represents an n-octadecyl radical, $G_{32}$ represents a reducing sugar residue and y represents a decimal number greater than or equal to 1.05 and less than or equal to 5, said composition ($C_{32}$) consisting of a mixture of compounds of formulae ($VIII_1$), ($VIII_2$), ($VIII_3$) , ($VIII_4$) and ($VIII_5$) :

$$R_{32}-O-(G_{32})_1-H \qquad (VIII_1),$$

$$R_{32}-O-(G_{32})_2-H \qquad (VIII_2) ,$$

$$R_{32}-O-(G_{32})_3-H \qquad (VIII_3),$$

$$R_{32}-O-(G_{32})_4-H \qquad (VIII_4),$$

$$R_{32}-O-(G_{32})_5-H \qquad (VIII_5),$$

in molar proportions of said compounds of formulae ($VIII_1$), ($VIII_2$), ($VIII_3$) , ($VIII_4$) and ($VIII_5$) respectively equal to $b'_1$, $b_2$, $b'_3$, $b'_4$ and $b'_5$, such that the sum $b'_1 + b'_2 + b'_3 + b'_4 + b'_5$ is equal to 1, and such that the sum $b'_1 + 2b'_2 + 3b'_3 + 4b'_4 + 5b'_5$ is equal to y,

it being understood that the sum of the mass proportions of compositions ($C_{31}$) and ($C_{32}$) is equal to 100%.

2. Composition (EM) as defined in Claim 1, **characterized in that**, in formulae (III), (IV), (V) and (VI), said reducing sugar residues $G_{21}$, $G_{22}$, $G_{23}$ and $G_{24}$ represent the same reducing sugar residue chosen from a glucose residue, a xylose residue and an arabinose residue.

3. Composition (EM) as defined in Claim 2, **characterized in that**, in formulae (III), (IV), (V) and (VI), said reducing sugar residues $G_{21}$, $G_{22}$, $G_{23}$ and $G_{24}$ represent a glucose residue.

4. Composition (EM) as defined in Claim 2, **characterized in that**, in formulae (III), (IV), (V) and (VI), said reducing sugar residues $G_{21}$, $G_{22}$, $G_{23}$ and $G_{24}$ represent a xylose residue.

5. Composition (EM) as defined in any one of Claims 1 to 4, **characterized in that**, in formulae (III), (IV), (V) and (VI), r, s, t and u respectively represent, independently of each other, a decimal number greater than or equal to 1.05 and less than or equal to 2.5.

6. Composition (EM) as defined in any one of Claims 1 to 5, **characterized in that**, in formulae (VII) and (VIII), said reducing sugar residues $G_{31}$ and $G_{32}$ represent the same reducing sugar residue chosen from a glucose residue, a xylose residue and an arabinose residue.

7. Composition (EM) as defined in Claim 6, **characterized in that**, in formulae (VII) and (VIII), said reducing sugar residues $G_{31}$ and $G_{32}$ represent a glucose residue.

8. Composition (EM) as defined in Claim 6, **characterized in that**, in formulae (VII) and (VIII), said reducing sugar residues $G_{31}$ and $G_{32}$ represent a xylose residue.

9. Composition (EM) as defined in any one of Claims 1 to 8, **characterized in that**, in formulae (VII) and (VIII), x and y respectively represent a decimal number greater than or equal to 1.05 and less than or equal to 2.5.

10. Composition (EM) as defined in any one of Claims 1 to 9, **characterized in that** the mass ratio:

$$\Delta = \text{Mass of composition } (C_2)/\text{Mass of composition } (C_3),$$

is greater than or equal to 1 and less than or equal to 10.

11. Composition (EM) as defined in any one of Claims 1 to 10, **characterized in that** the diluent is 1,3-propanediol or glycerol.

12. Composition (EM) as claimed in any one of Claims 1 to 11, **characterized in that** it also comprises:
3) - Up to 7% by mass of water,
it being understood that the sum of the mass proportions of said diluent, of said composition (C) and of water is equal to 100%.

13. Process for preparing the composition (EM) as defined in any one of Claims 1 to 12, comprising the following successive steps:

- A <u>step A)</u> of reacting, in the desired proportions, a reducing sugar of formula (IX) or a mixture of reducing sugars of formula (IX):

HO-(G)-H        (IX)

in which G represents the reducing sugar residue, with a molar excess of a mixture of alcohols of formula (II):

$C_mH_{2m+1}$-OH        (II),

as defined previously, to form a mixture of compounds of formulae (III), (IV), (V), (VI), (VII) and (VIII) and an excess of said mixture of alcohols of formula (II),
- A <u>step B)</u> of partial removal of the excess of said mixture of alcohols of formula (II) to form said composition (C) as defined previously,
- a <u>step C)</u> of mixing said composition (C) with at least one diluent as defined previously, and, if necessary or if desired,
- a <u>step D</u> of adding water.

14. Use of a composition (EM) as defined in any one of Claims 1 to 12, as an emulsifier for the preparation of a fluid emulsion of oil-in-water type for topical use.

15. Emulsion of oil-in-water type (E), comprising:

- from 50% to 80% by mass of at least one cosmetically acceptable aqueous phase ($P_1$),
- from 0.5% to 15% by mass of at least one composition (EM) as defined in any one of Claims 1 to 12, and
- from 5% to 49.5% by mass of at least one fatty phase ($P_2$) ,

it being understood that the sum of the mass proportions of said aqueous phase ($P_1$), of said composition (EM) and of said fatty phase ($P_2$) is equal to 100%.

## RÉFÉRENCES CITÉES DANS LA DESCRIPTION

**Documents brevets cités dans la description**

- FR 2668080 A1
- WO 9637285 A
- WO 9513863 A
- WO 0056438 A1
- FR 2807435 A1
- WO 2005110588 A1
- WO 2007113440 A1
- EP 0971683 A
- WO 9936445 A
- WO 2005040230 A

**Littérature non-brevet citée dans la description**

- **DANIEL VOET ; JUDITH G. VOET.** Biochemistry. John Wyley & Sons, 1990, 250
- PARLEAM - POLYSYNLANE™. **MICHEL ; IRENE ASH.** Thesaurus of Chemical Products. Chemical Publishing Co, Inc, 1986, vol. I, 211